# EUROPÄISCHE PATENTSCHRIFT

(11) **EP 1 341 922 B1**
(45) Veröffentlichungstag und Bekanntmachung des Hinweises auf die Patenterteilung: **02.03.2005**
(21) Anmeldenummer: 01985874.5
(22) Anmeldetag: 12.12.2001
(51) Int. Cl.: C12N 15/81

(54) **HEFESTAMM ZUR PRÜFUNG DER GENO- UND ZYTOTOXIZITÄT KOMPLEXER UMWELTKONTAMINATIONEN**
YEAST STRAIN FOR TESTING THE GENO- AND CYTOTOXICITY OF COMPLEX ENVIRONMENTAL CONTAMINATION
SOUCHES DE LEVURE POUR LE CONTROLE DE LA GENOTOXICITE ET DE LA CYTOTOXICITE DE CONTAMINATIONS DE L'ENVIRONNEMENT COMPLEXES

(30) Priorität: 12.12.2000 DE 10061872
(43) Veröffentlichungstag der Anmeldung: 10.09.2003
(73) Patentinhaber: Lichtenberg-Fraté, Hella, 53129 Bonn (DE)
(72) Erfinder: Lichtenberg-Fraté, Hella, 53129 Bonn (DE)
(74) Vertreter: Helbing, Jörg, Dr. Dipl.-Chem.
(86) Internationale Anmeldenummer: PCT/EP2001/014610
(87) Internationale Veröffentlichungsnummer: WO 2002/048338

(56) Entgegenhaltungen:
- WO-A-99/53092
- DE-A- 19 549 417
- BILLINTON N. ET AL: "Development of a green fluorescent protein reporter for a yeast gentoxicity biosensor", BIOSENSORS AND BIOELECTRONICS, , 01. Oktober 1998, Band 13, Nr. 7-8, Seiten 831 - 838

### SEQUENZPROTOKOLL

<110> Lichtenberg-Fraté, Hella
<120> Hefestamm zur Prüfung der Geno- und Zytotoxizität
   komplexer Umweltkontaminationen
<130> 013066wo/JH/ml
<140>
   <141>
<160> 24
<170> PatentIn Ver. 2.1
<210> 1
   <211> 1275
   <212> DNA
   <213> Saccharomyces cerevisiae
<220>
   <221> promoter
   <222> Complement((1)..(1275))
<400> 1
   cagttataag gaaatatata tggtaccttg aaatagagct aagttctgag cgtttgattt 60
   ttctatactt tcgtagcagc tttagtgaag aaagagaaaa gttgctgctc tagattttgt 120
   atcggctatt acgtcaagtg agaagagttt tggccttcgc ttcagttaga tcttctatta 180
   tttccttttt tttctttttg tttgtaattg ttttttattt ttttttttgc gcgaaacttt 240
   gctatattgg gtaacgcgta aaatactttt tattattgca gtaaggcgga agggtcttcc 300
   cctttgcatg ttaaatagca tacatggcac cactcaggtc cagaacgtga cacatctttg 360
   caccacttgt gtattttcaa gatgttaaat ttttgataca taagctttat catgcaggtg 420
   cagtagacgc atttgcgtac gcgttgagta gtaatgcaag aacgcaacaa tttcacgctt 480
   tctacttaag aggacagatc ttcgagcaga atatttttct cctagcgcgg ctttcagaat 540
   cttcttacca tctgcaggac atcacatgca agcacatgaa gctggaacca cgcaccatta 600
   aatgtaaacc tacagacata gctagcacag aaatgctact ttggaaacct cggttgcatc 660
   ggctgacctc ggctgttcag caacgtgagc tggttgttgt tattgttcat agaatcctgt 720
   attttaccag tgtagctttg tccgcccacc ccacgctttt ggccactagt atgtacgtat 780
   atgcgtgggc tctatatcat tgaaaacgaa ttttccacgc aaaacccact tcacaccata 840
   aaggccttat gacgatgtac attcttcccc tcccccctcc tcccgaagga acccttatag 900
   tgatcctaat acggtataac gtaaaccgga gttgtcagca gagagaagcg aataatatgt 960
   tttgcgtggg gttcccacga gcggcaggaa ccgtagcgca ttgaaaaatg aatcatcgat 1020
   catctttggg tttcaagggt tcggacttcg ccaaaggtcc gaatacttcc accagtattt 1080
   ctctttttcc ctttccagtc tcgttcttcg ccgaatcccc gctttcccat catgatcgtt 1140
   tctctgtttc tctctttccg gaacaggctc ttttctgacc gccagttcgt gcggttagtt 1200
   ctgtggatct tgcaggcact tgccggttta gccacacttc tccgctgttt gtgtcgaatt 1260
   tccctcgagt atgag 1275
<210> 2
   <211> 345
   <212> DNA
   <213> Saccharomyces cerevisiae
<220>
   <221> promoter
   <222> (1)..(345)
<400> 2
   ttatttatga aaattggcct gtaaatatat acataaataa atatatgcgt caagctcttt 60
   tattcggttt gtccagtaat cctcttacaa tatatatata tatatatacg catgtagcgt 120
   attcaagaac attacattta aaaaatagag ggtagaacaa taaaaatgca cttaacaagt 180
   tccgtaagta gaaattataa tcagtctaag tacaaattta taccttagct tcatttcagt 240
   taagggcgat gcaataaaag tggaagaaaa aaaaagaagg gaagaggttt gataatatat 300
   atatatatat atatataaca ttgctcagag aaacatacat cagtg 345
<210> 3
   <211> 438
   <212> DNA
   <213> Saccharomyces cerevisiae
<220>
   <221> promoter
   <222> (1)..(438)
<400> 3
   taaagtgacc tggctctata gtgttgtccc tctcgcgagg accattgttg cttgcatatg 60
   gcttgaaaca tatgtcatca catctgagcg attttacctc ttagaattag tttagatata 120
   tatgagttga tgaataaata gttataaaaa cttgctttgg cttcgatata tgaccgttat 180
   ttttgactaa gttttaacga aggaatctaa cctcgttctt gtaattacca aaatcttcaa 240
   caacgcgctg ttggaggtat ctctatggat gtggcttgaa atatggatgt cttgcctact 300
   tctacttctg ggaaaggcat ttttactcga tcgcgttaat atatgcatca agaaaataaa 360
   aaataaaacg cgaagagcta aaaaaaaaaa agaaaaccta ctataaataa ccgattagaa 420
   tcgagttttt gtattgaa                                               438
<210> 4
   <211> 209
   <212> DNA
   <213> Saccharomyces cerevisiae
<220>
   <221> promoter
   <222> (1)..(209)
<400> 4
   cttttttttt ttttttttat agcacgcaac tgaaaaaaaa aaaaagaaaa atttttcatc 60
   ttcgctcgac gtttcttttg tagtactcat ctctttttat ataaagatta attagttatt 120
   gtcgctttgc ttttccttct ttaaaaaatg tttcttgctt ttggattttc agatgtccca 180
   agatcattac agtattttaa ttgaacaaa                                   209
<210> 5
   <211> 361
   <212> DNA
   <213> Saccharomyces cerevisiae
<220>
   <221> promoter
   <222> (1)..(361)
<400> 5
   tatcacattc cggagtgtca ccccccctct ctcaacacag taatccataa accagtttta 60
   catacacgta aaaaagaaca ggaataaagc ttaatcggat tattaactca tacgcttgtc 120
   acatattgtt cgaacaattc tggttctttc gagtttcgca gaactttttg aatttttctt 180
   ttttttctag aacgccgtgg aagaaaaaca cgcgcatggt tttatgagcg gttaattctc 240
   atcttaatac caaccaggtc cttccgccac cccctaaaac atataaatat gcagcttatc 300
   ccttcaattc ttaacatctg tgacctcctc atttcttccc gctgtattag agttcaagaa 360
   a                                                                 361
<210> 6
   <211> 358
   <212> DNA
   <213> Saccharomyces cerevisiae
<220>
   <221> promoter
   <222> (1)..(358)
<400> 6
   cacaattgca tacttttcca ttataactga ctgtttcaga tcctgcaata gaaagtattt 60
   tttaagtaat gaaatagtgc ttccattgat agtaagtgag taataagacg atcggaaact 120
   attaatataa gtatagttta catatatata tatatatatg tgccagacga taatatcacc 180
   gtagtttccg gccgggttat gccacgcgaa aacgcctcga gccaagggaa aaactaaact 240
   acaaaacaaa acaaaacaaa acaaaaacat ctactatatc gttttaccca attcagtata 300
   tccgtctaca acagttctcg ccacccaaga tctgtaaact tacaactgca aacaaaca   358
<210> 7
   <211> 559
   <212> DNA
   <213> Saccharomyces cerevisiae
<220>
   <221> promoter
   <222> (1)..(559)
<400> 7
   ggtnacctca cgtcatggaa attttcgcct tattcatgtt gttgggtatc ttcacaacct 60
   tgttgatccc agaaactaag agaaagactc tagaagaaat taacgagcta taccacgatg 120
   aaatcgatcc tgctacgcta aacttcagaa acaagaataa tgacattgaa tcttccagcc 180
   catctcaact tcaacatgaa gcataaaagc ctcaaagatg cactaaaact tgtaaactag 240
   aacaaataat acaaaaacat ttttataaac ttattatcaa accccttaca taatctataa 300
   atactgtcag gttacatatt tattcgataa tttcttttaa tttcattatt tcctcacatc 360
   tctctgccat cctgttggct ggtgccagag cagagcatat cgtcctttct tttttagttc 420
   cagacgttac ccgacatatc atttctcgag cctctggaaa ccacgaaacg ttttacaaat 480
   tgcacatcta aaagaaatat aaacacagat caggtatctc ataaagtaca ttaatcgact 540
   aagcaagcga cttgagaca                                              559
<210> 8
   <211> 939
   <212> DNA
   <213> Saccharomyces cerevisiae
<220>
   <221> promoter
   <222> (1)..(939)
<400> 8
   aagcttcctg aaacggagaa acataaacag gcattgctgg gatcacccat acatcactct 60
   gttttgcctg accttttccg gtaatttgaa aacaaacccg gtctcgaagc ggagatccgg 120
   cgataattac cgcagaaata aacccataca cgagacgtag aaccagccgc acatggccgg 180
   agaaactcct gcgagaattt cgtaaactcg cgcgcattgc atctgtattt cctaatgcgg 240
   cacttccagg cctcgagacc tctgacatgc ttttgacagg aatagacatt ttcagaatgt 300
   tatccatatg cctttcgggt ttttttcctt ccttttccat catgaaaaat ctctcgagac 360
   cgtttatcca ttgctttttt gttgtctttt tccctcgttc acagaaagtc tgaagaagct 420
   atagtagaac tatgagcttt ttttgtttct gttttccttt tttttttttt tacctctgtg 480
   gaaattgtta ctctcacact ctttagttcg tttgtttgtt ttgtttattc caattatgac 540
   cggtgacgaa acgtggtcca tggtgggtac cgcttatgct cccctccatt agtttcgatt 600
   atataaaaag gccaaatatt gtgttatttt caaatgtcct atcattatcg tctaacatct 660
   aatttctctt aaattttttc tctttctttc ctataacacc aatagtgaaa atcttttttt 720
   cttctatatc tacaaaaact ttttttttct atcaacctcg ttgataaatt ttttctttaa 780
   caatcgttaa taattaatta attggaaaat aaccattttt tctctctttt atacacacat 840
   tcaaaagaaa gaaaaaaaat ataccccagc tagttaaaga aaatcattga aaagaataag 900
   aagataagaa agatttaatt atcaaacaat atcaatatg                        939
<210> 9
   <211> 738
   <212> DNA
   <213> Saccharomyces cerevisiae
<220>
   <221> promoter
   <222> (1)..(738)
<400> 9
   caataatcca tcatatacca ttaccctgat tcccatcgaa gaaaaggcgg tgtcccctta 60
   cccgtccgct catgccaaga gattaattca taaccgctct tccttggatc agaagtgaac 120
   atatgaagtt gcaactacta catacttact accgtagtcc atcatcaagg acccaaacat 180
   tcacgactcc agcgcgccac gttcttcgcc atactgctta acattttggt acgagtgcga 240
   attagggaag tcgatgataa aatagaatat gcgaaaaaga ggaagagcag ccgtgagaaa 300
   aaagaaaaaa aaaggcctaa ggtattctct actccaaaat cgtcgaggga gggcaaaaga 360
   aatttttttt gtttaaggga attgcgaagt caattgattg atgaagtagc ttctctggaa 420
   aaatggcgga cagacgctgc tcaaataacc gtcgaagggg gaaaggttat cggtggaaac 480
   atatttgtaa gtacataata gtgaatttac ttcttgtcgg cgtcccctaa acgtaattgg 540
   cggtgtgatg gtacttcgtt atataaaggt gtgtaatatc ctcttttacc atctattatt 600
   tcttccagca tttcttgctg gataacctac tgtatccaag ctactgggct tttttaaaca 660
   tacccataac tttttttttt ttcatttttc gttgctgtgt gctagtacaa tttaagcaaa 720
   aggaaactgt tttgcgtt                                               738
<210> 10
   <211> 736
   <212> DNA
   <213> Aequorea victoria
<220>
   <221> CDS
   <222> (14)..(727)
<400> 10
   aagctttatt aaa atg tct aaa ggt gaa gaa tta ttc act ggt gtt gtc     49
                  Met Ser Lys Gly Glu Glu Leu Phe Thr Gly Val Val
                   1               5                  10
   cca att ttg gtt gaa tta gat ggt gat gtt aat ggt cac aaa ttt tct    97
   Pro Ile Leu Val Glu Leu Asp Gly Asp Val Asn Gly His Lys Phe Ser
           15                  20                  25
   gtc tcc ggt gaa ggt gaa ggt gat gct act tac ggt aaa ttg acc tta    145
   Val Ser Gly Glu Gly Glu Gly Asp Ala Thr Tyr Gly Lys Leu Thr Leu
       30                  35                  40
   aaa ttt att tgt act act ggt aaa ttg cca gtt cca tgg cca acc tta    193
   Lys Phe Ile Cys Thr Thr Gly Lys Leu Pro Val Pro Trp Pro Thr Leu
   45                  50                  55                  60
   gtc act act ttc ggt tat ggt gtt caa tgt ttt gct aga tac cca gat    241
   Val Thr Thr Phe Gly Tyr Gly Val Gln Cys Phe Ala Arg Tyr Pro Asp
                   65                  70                  75
   cat atg aaa caa cat gac ttt ttc aag tct gcc atg cca gaa ggt tat    289
   His Met Lys Gln His Asp Phe Phe Lys Ser Ala Met Pro Glu Gly Tyr
               80                  85                  90
   gtt caa gaa aga act att ttt ttc aaa gat gac ggt aac tac aag acc    337
   Val Gln Glu Arg Thr Ile Phe Phe Lys Asp Asp Gly Asn Tyr Lys Thr
           95                  100                 105
   aga gct gaa gtc aag ttt gaa ggt gat acc tta gtt aat aga atc gaa    385
   Arg Ala Glu Val Lys Phe Glu Gly Asp Thr Leu Val Asn Arg Ile Glu
       110                 115                 120
   tta aaa ggt att gat ttt aaa gaa gat ggt aac att tta ggt cac aaa    433
   Leu Lys Gly Ile Asp Phe Lys Glu Asp Gly Asn Ile Leu Gly His Lys
   125                 130                 135                 140
   ttg gaa tac aac tat aac tct cac aat gtt tac atc atg gct gac aaa    481
   Leu Glu Tyr Asn Tyr Asn Ser His Asn Val Tyr Ile Met Ala Asp Lys
                   145                 150                 155
   caa aag aat ggt atc aaa gtt aac ttc aaa att aga cac aac att gaa    529
   Gln Lys Asn Gly Ile Lys Val Asn Phe Lys Ile Arg His Asn Ile Glu
               160                 165                 170
   gat ggt tct gtt caa tta gct gac cat tat caa caa aat act cca att    577
   Asp Gly Ser Val Gln Leu Ala Asp His Tyr Gln Gln Asn Thr Pro Ile
           175                 180                 185
   ggt gat ggt cca gtc ttg tta cca gac aac cat tac tta tcc act caa    625
   Gly Asp Gly Pro Val Leu Leu Pro Asp Asn His Tyr Leu Ser Thr Gln
       190                 195                 200
   tct gcc tta tcc aaa gat cca aac gaa aag aga gac cac atg gtc ttg    673
   Ser Ala Leu Ser Lys Asp Pro Asn Glu Lys Arg Asp His Met Val Leu
   205                 210                 215                 220
   tta gaa ttt gtt act gct gct ggt att acc cat ggt atg gat gaa ttg    721
   Leu Glu Phe Val Thr Ala Ala Gly Ile Thr His Gly Met Asp Glu Leu
                   225                 230                 235
   tac aaa taactgcag                                                  736
   Tyr Lys
<210> 11
   <211> 238
   <212> PRT
   <213> Aequorea victoria
<400> 11
   Met Ser Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Phe
       50                  55                  60
   Gly Tyr Gly Val Gln Cys Phe Ala Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 12
   <211> 859
   <212> DNA
   <213> Discosoma sp.
<220>
   <221> CDS
   <222> (54)..(728)
<400> 12
   gtttcagcca gtgacggtca gtgacagggt gagccacttg gtataccaac aaa atg      56
                                                              Met
                                                              1
   agg tct tcc aag aat gtt atc aag gag ttc atg agg ttt aag gtt cgc    104
   Arg Ser Ser Lys Asn Val Ile Lys Glu Phe Met Arg Phe Lys Val Arg
               5                   10                  15
   atg gaa gga acg gtc aat ggg cac gag ttt gaa ata gaa ggc gaa gga    152
   Met Glu Gly Thr Val Asn Gly His Glu Phe Glu Ile Glu Gly Glu Gly
           20                  25                  30
   gag ggg agg cca tac gaa ggc cac aat acc gta aag ctt aag gta acc    200
   Glu Gly Arg Pro Tyr Glu Gly His Asn Thr Val Lys Leu Lys Val Thr
       35                  40                  45
   aag ggg gga cct ttg cca ttt gct tgg gat att ttg tca cca caa ttt    248
   Lys Gly Gly Pro Leu Pro Phe Ala Trp Asp Ile Leu Ser Pro Gln Phe
   50                  55                  60                  65
   cag tat gga agc aag gta tat gtc aag cac cct gcc gac ata cca gac    296
   Gln Tyr Gly Ser Lys Val Tyr Val Lys His Pro Ala Asp Ile Pro Asp
                   70                  75                  80
   tat aaa aag ctg tca ttt cct gaa gga ttt aaa tgg gaa agg gtc atg    344
   Tyr Lys Lys Leu Ser Phe Pro Glu Gly Phe Lys Trp Glu Arg Val Met
               85                  90                  95
   aac ttt gaa gac ggt ggc gtc gtt act gta acc cag gat tcc agt ttg    392
   Asn Phe Glu Asp Gly Gly Val Val Thr Val Thr Gln Asp Ser Ser Leu
           100                 105                 110
   cag gat ggc tgt ttc atc tac aag gtc aag ttc att ggc gtg aac ttt    440
   Gln Asp Gly Cys Phe Ile Tyr Lys Val Lys Phe Ile Gly Val Asn Phe
       115                 120                 125
   cct tcc gat gga cct gtt atg caa aag aag aca atg ggc tgg gaa gcc    488
   Pro Ser Asp Gly Pro Val Met Gln Lys Lys Thr Met Gly Trp Glu Ala
   130                 135                 140                 145
   agc act gag cgt ttg tat cct cgt gat ggc gtg ttg aaa gga gag att    536
   Ser Thr Glu Arg Leu Tyr Pro Arg Asp Gly Val Leu Lys Gly Glu Ile
                   150                 155                 160
   cat aag gct ctg aag ctg aaa gac ggt ggt cat tac cta gtt gaa ttc    584
   His Lys Ala Leu Lys Leu Lys Asp Gly Gly His Tyr Leu Val Glu Phe
               165                 170                 175
   aaa agt att tac atg gca aag aag cct gtg cag cta cca ggg tac tac    632
   Lys Ser Ile Tyr Met Ala Lys Lys Pro Val Gln Leu Pro Gly Tyr Tyr
           180                 185                 190
   tat gtt gac tcc aaa ctg gat ata aca agc cac aac gaa gac tat aca    680
   Tyr Val Asp Ser Lys Leu Asp Ile Thr Ser His Asn Glu Asp Tyr Thr
       195                 200                 205
   atc gtt gag cag tat gaa aga acc gag gga cgc cac cat ctg ttc ctt    728
   Ile Val Glu Gln Tyr Glu Arg Thr Glu Gly Arg His His Leu Phe Leu
   210                 215                 220                 225
   taaggctgaa cttggctcag acgtgggtga gcggtaatga ccacaaaagg cagcgaagaa 788
   aaaccatgat cgtttttttt aggttggcag cctgaaatcg taggaaatac atcagaaatg 848
   ttacaaacag g                                                      859
<210> 13
   <211> 225
   <212> PRT
   <213> *Discosoma sp.*
<400> 13
   Met Arg Ser Ser Lys Asn Val Ile Lys Glu Phe Met Arg Phe Lys Val
   1               5                   10                  15
   Arg Met Glu Gly Thr Val Asn Gly His Glu Phe Glu Ile Glu Gly Glu
               20                  25                  30
   Gly Glu Gly Arg Pro Tyr Glu Gly His Asn Thr Val Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Val Tyr Val Lys His Pro Ala Asp Ile Pro
   65                  70                  75                  80
   Asp Tyr Lys Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Cys Phe Ile Tyr Lys Val Lys Phe Ile Gly Val Asn
           115                 120                 125
   Phe Pro Ser Asp Gly Pro Val Met Gln Lys Lys Thr Met Gly Trp Glu
       130                 135                 140
   Ala Ser Thr Glu Arg Leu Tyr Pro Arg Asp Gly Val Leu Lys Gly Glu
   145                 150                 155                 160
   Ile His Lys Ala Leu Lys Leu Lys Asp Gly Gly His Tyr Leu Val Glu
                   165                 170                 175
   Phe Lys Ser Ile Tyr Met Ala Lys Lys Pro Val Gln Leu Pro Gly Tyr
               180                 185                 190
   Tyr Tyr Val Asp Ser Lys Leu Asp Ile Thr Ser His Asn Glu Asp Tyr
           195                 200                 205
   Thr Ile Val Glu Gln Tyr Glu Arg Thr Glu Gly Arg His His Leu Phe
       210                 215                 220
   Leu
   225
<210> 14
   <211> 1166
   <212> DNA
   <213> Saccharomyces cerevisiae
<220>
   <221> CDS
   <222> (223)..(1023)
<400> 14
   agcttttcaa ttcatctttt ttttttttgt tctttttttt gattccggtt tctttgaaat 60
   ttttttgatt cggtaatctc cgagcagaag gaagaacgaa ggaaggagca cagacttaga 120
   ttggtatata tacgcatatg tggtgttgaa gaaacatgaa attgcccagt attcttaacc 180
   caactgcaca gaacaaaaac ctgcaggaaa cgaagataaa tc atg tcg aaa gct    234
                                                  Met Ser Lys Ala
                                                   1
   aca tat aag gaa cgt gct gct act cat cct agt cct gtt gct gcc aag   282
   Thr Tyr Lys Glu Arg Ala Ala Thr His Pro Ser Pro Val Ala Ala Lys
    5                  10                  15                  20
   cta ttt aat atc atg cac gaa aag caa aca aac ttg tgt gct tca ttg   330
   Leu Phe Asn Ile Met His Glu Lys Gln Thr Asn Leu Cys Ala Ser Leu
                   25                  30                  35
   gat gtt cgt acc acc aag gaa tta ctg gag tta gtt gaa gca tta ggt   378
   Asp Val Arg Thr Thr Lys Glu Leu Leu Glu Leu Val Glu Ala Leu Gly
               40                  45                  50
   ccc aaa att tgt tta cta aaa aca cat gtg gat atc ttg act gat ttt   426
   Pro Lys Ile Cys Leu Leu Lys Thr His Val Asp Ile Leu Thr Asp Phe
           55                  60                  65
   tcc atg gag ggc aca gtt aag ccg cta aag gca tta tcc gcc aag tac   474
   Ser Met Glu Gly Thr Val Lys Pro Leu Lys Ala Leu Ser Ala Lys Tyr
       70                  75                  80
   aat ttt tta ctc ttc gaa gac aga aaa ttt gct gac att ggt aat aca   522
   Asn Phe Leu Leu Phe Glu Asp Arg Lys Phe Ala Asp Ile Gly Asn Thr
   85                  90                  95                  100
   gtc aaa ttg cag tac tct gcg ggt gta tac aga ata gca gaa tgg gca   570
   Val Lys Leu Gln Tyr Ser Ala Gly Val Tyr Arg Ile Ala Glu Trp Ala
                   105                 110                 115
   gac att acg aat gca cac ggt gtg gtg ggc cca ggt att gtt agc ggt   618
   Asp Ile Thr Asn Ala His Gly Val Val Gly Pro Gly Ile Val Ser Gly
               120                 125                 130
   ttg aag cag gcg gcg gaa gaa gta aca aag gaa cct aga ggc ctt ttg     666
   Leu Lys Gln Ala Ala Glu Glu Val Thr Lys Glu Pro Arg Gly Leu Leu
           135                 140                 145
   atg tta gca gaa ttg tca tgc aag ggc tcc cta gct act gga gaa tat     714
   Met Leu Ala Glu Leu Ser Cys Lys Gly Ser Leu Ala Thr Gly Glu Tyr
       150                 155                 160
   act aag ggt act gtt gac att gcg aag agc gac aaa gat ttt gtt atc     762
   Thr Lys Gly Thr Val Asp Ile Ala Lys Ser Asp Lys Asp Phe Val Ile
   165                 170                 175                 180
   ggc ttt att gct caa aga gac atg ggt gga aga gat gaa ggt tac gat     810
   Gly Phe Ile Ala Gln Arg Asp Met Gly Gly Arg Asp Glu Gly Tyr Asp
                   185                 190                 195
   tgg ttg att atg aca ccc ggt gtg ggt tta gat gac aag gga gac gca     858
   Trp Leu Ile Met Thr Pro Gly Val Gly Leu Asp Asp Lys Gly Asp Ala
               200                 205                 210
   ttg ggt caa cag tat aga acc gtg gat gat gtg gtc tct aca gga tct     906
   Leu Gly Gln Gln Tyr Arg Thr Val Asp Asp Val Val Ser Thr Gly Ser
           215                 220                 225
   gac att att att gtt gga aga gga cta ttt gca aag gga agg gat gct     954
   Asp Ile Ile Ile Val Gly Arg Gly Leu Phe Ala Lys Gly Arg Asp Ala
       230                 235                 240
   aag gta gag ggt gaa cgt tac aga aaa gca ggc tgg gaa gca tat ttg     1002
   Lys Val Glu Gly Glu Arg Tyr Arg Lys Ala Gly Trp Glu Ala Tyr Leu
   245                 250                 255                 260
   aga aga tgc ggc cag caa aac taaaaaactg tattataagt aaatgcatgt      1053
   Arg Arg Cys Gly Gln Gln Asn
                   265
   atactaaact cacaaattag agcttcaatt taattatatc agttattacc cgggaatctc 1113
   ggtcgtaatg atttctataa tgacgaaaaa aaaaaaattg gaaagaaaaa gct        1166
<210> 15
   <211> 267
   <212> PRT
   <213> Saccharomyces cerevisiae
<400> 15
   Met Ser Lys Ala Thr Tyr Lys Glu Arg Ala Ala Thr His Pro Ser Pro
   1               5                   10                  15
   Val Ala Ala Lys Leu Phe Asn Ile Met His Glu Lys Gln Thr Asn Leu
               20                  25                  30
   Cys Ala Ser Leu Asp Val Arg Thr Thr Lys Glu Leu Leu Glu Leu Val
           35                  40                  45
   Glu Ala Leu Gly Pro Lys Ile Cys Leu Leu Lys Thr His Val Asp Ile
       50                  55                  60
   Leu Thr Asp Phe Ser Met Glu Gly Thr Val Lys Pro Leu Lys Ala Leu
   65                  70                  75                  80
   Ser Ala Lys Tyr Asn Phe Leu Leu Phe Glu Asp Arg Lys Phe Ala Asp
                   85                  90                  95
   Ile Gly Asn Thr Val Lys Leu Gln Tyr Ser Ala Gly Val Tyr Arg Ile
               100                 105                 110
   Ala Glu Trp Ala Asp Ile Thr Asn Ala His Gly Val Val Gly Pro Gly
           115                 120                 125
   Ile Val Ser Gly Leu Lys Gln Ala Ala Glu Glu Val Thr Lys Glu Pro
       130                 135                 140
   Arg Gly Leu Leu Met Leu Ala Glu Leu Ser Cys Lys Gly Ser Leu Ala
   145                 150                 155                 160
   Thr Gly Glu Tyr Thr Lys Gly Thr Val Asp Ile Ala Lys Ser Asp Lys
                   165                 170                 175
   Asp Phe Val Ile Gly Phe Ile Ala Gln Arg Asp Met Gly Gly Arg Asp
               180                 185                 190
   Glu Gly Tyr Asp Trp Leu Ile Met Thr Pro Gly Val Gly Leu Asp Asp
           195                 200                 205
   Lys Gly Asp Ala Leu Gly Gln Gln Tyr Arg Thr Val Asp Asp Val Val
       210                 215                 220
   Ser Thr Gly Ser Asp Ile Ile Ile Val Gly Arg Gly Leu Phe Ala Lys
   225                 230                 235                 240
   Gly Arg Asp Ala Lys Val Glu Gly Glu Arg Tyr Arg Lys Ala Gly Trp
                   245                 250                 255
   Glu Ala Tyr Leu Arg Arg Cys Gly Gln Gln Asn
               260                 265
<210> 16
   <211> 4692
   <212> DNA
   <213> Künstliche Sequenz
<220>
   <223> Beschreibung der künstlichen Sequenz: Vektor pDsRed1-N1
<400> 16
   tagttattaa tagtaatcaa ttacggggtc attagttcat agcccatata tggagttccg 60
   cgttacataa cttacggtaa atggcccgcc tggctgaccg cccaacgacc cccgcccatt 120
   gacgtcaata atgacgtatg ttcccatagt aacgccaata gggactttcc attgacgtca 180
   atgggtggag tatttacggt aaactgccca cttggcagta catcaagtgt atcatatgcc 240
   aagtacgccc cctattgacg tcaatgacgg taaatggccc gcctggcatt atgcccagta 300
   catgacctta tgggactttc ctacttggca gtacatctac gtattagtca tcgctattac 360
   catggtgatg cggttttggc agtacatcaa tgggcgtgga tagcggtttg actcacgggg 420
   atttccaagt ctccacccca ttgacgtcaa tgggagtttg ttttggcacc aaaatcaacg 480
   ggactttcca aaatgtcgta acaactccgc cccattgacg caaatgggcg gtaggcgtgt 540
   acggtgggag gtctatataa gcagagctgg tttagtgaac cgtcagatcc gctagcgcta 600
   ccggactcag atctcgagct caagcttcga attctgcagt cgacggtacc gcgggcccgg 660
   gatccaccgg tcgccaccat ggtgcgctcc tccaagaacg tcatcaagga gttcatgcgc 720
   ttcaaggtgc gcatggaggg caccgtgaac ggccacgagt tcgagatcga gggcgagggc 780
   gagggccgcc cctacgaggg ccacaacacc gtgaagctga aggtgaccaa gggcggcccc 840
   ctgcccttcg cctgggacat cctgtccccc cagttccagt acggctccaa ggtgtacgtg 900
   aagcaccccg ccgacatccc cgactacaag aagctgtcct tccccgaggg cttcaagtgg 960
   gagcgcgtga tgaacttcga ggacggcggc gtggtgaccg tgacccagga ctcctccctg 1020
   caggacggct gcttcatcta caaggtgaag ttcatcggcg tgaacttccc ctccgacggc 1080
   cccgtaatgc agaagaagac catgggctgg gaggcctcca ccgagcgcct gtacccccgc 1140
   gacggcgtgc tgaagggcga gatccacaag gccctgaagc tgaaggacgg cggccactac 1200
   ctggtggagt tcaagtccat ctacatggcc aagaagcccg tgcagctgcc cggctactac 1260
   tacgtggact ccaagctgga catcacctcc cacaacgagg actacaccat cgtggagcag 1320
   tacgagcgca ccgagggccg ccaccacctg ttcctgtagc ggccgcgact ctagatcata 1380
   atcagccata ccacatttgt agaggtttta cttgctttaa aaaacctccc acacctcccc 1440
   ctgaacctga aacataaaat gaatgcaatt gttgttgtta acttgtttat tgcagcttat 1500
   aatggttaca aataaagcaa tagcatcaca aatttcacaa ataaagcatt tttttcactg 1560
   cattctagtt gtggtttgtc caaactcatc aatgtatctt aaggcgtaaa ttgtaagcgt 1620
   taatattttg ttaaaattcg cgttaaattt ttgttaaatc agctcatttt ttaaccaata 1680
   ggccgaaatc ggcaaaatcc cttataaatc aaaagaatag accgagatag ggttgagtgt 1740
   tgttccagtt tggaacaaga gtccactatt aaagaacgtg gactccaacg tcaaagggcg 1800
   aaaaaccgtc tatcagggcg atggcccact acgtgaacca tcaccctaat caagtttttt 1860
   ggggtcgagg tgccgtaaag cactaaatcg gaaccctaaa gggagccccc gatttagagc 1920
   ttgacgggga aagccggcga acgtggcgag aaaggaaggg aagaaagcga aaggagcggg 1980
   cgctagggcg ctggcaagtg tagcggtcac gctgcgcgta accaccacac ccgccgcgct 2040
   taatgcgccg ctacagggcg cgtcaggtgg cacttttcgg ggaaatgtgc gcggaacccc 2100
   tatttgttta tttttctaaa tacattcaaa tatgtatccg ctcatgagac aataaccctg 2160
   ataaatgctt caataatatt gaaaaaggaa gagtcctgag gcggaaagaa ccagctgtgg 2220
   aatgtgtgtc agttagggtg tggaaagtcc ccaggctccc cagcaggcag aagtatgcaa 2280
   agcatgcatc tcaattagtc agcaaccagg tgtggaaagt ccccaggctc cccagcaggc 2340
   agaagtatgc aaagcatgca tctcaattag tcagcaacca tagtcccgcc cctaactccg 2400
   cccatcccgc ccctaactcc gcccagttcc gcccattctc cgccccatgg ctgactaatt 2460
   ttttttattt atgcagaggc cgaggccgcc tcggcctctg agctattcca gaagtagtga 2520
   ggaggctttt ttggaggcct aggcttttgc aaagatcgat caagagacag gatgaggatc 2580
   gtttcgcatg attgaacaag atggattgca cgcaggttct ccggccgctt gggtggagag 2640
   gctattcggc tatgactggg cacaacagac aatcggctgc tctgatgccg ccgtgttccg 2700
   gctgtcagcg caggggcgcc cggttctttt tgtcaagacc gacctgtccg gtgccctgaa 2760
   tgaactgcaa gacgaggcag cgcggctatc gtggctggcc acgacgggcg ttccttgcgc 2820
   agctgtgctc gacgttgtca ctgaagcggg aagggactgg ctgctattgg gcgaagtgcc 2880
   ggggcaggat ctcctgtcat ctcaccttgc tcctgccgag aaagtatcca tcatggctga 2940
   tgcaatgcgg cggctgcata cgcttgatcc ggctacctgc ccattcgacc accaagcgaa 3000
   acatcgcatc gagcgagcac gtactcggat ggaagccggt cttgtcgatc aggatgatct 3060
   ggacgaagag catcaggggc tcgcgccagc cgaactgttc gccaggctca aggcgagcat 3120
   gcccgacggc gaggatctcg tcgtgaccca tggcgatgcc tgcttgccga atatcatggt 3180
   ggaaaatggc cgcttttctg gattcatcga ctgtggccgg ctgggtgtgg cggaccgcta 3240
   tcaggacata gcgttggcta cccgtgatat tgctgaagag cttggcggcg aatgggctga 3300
   ccgcttcctc gtgctttacg gtatcgccgc tcccgattcg cagcgcatcg ccttctatcg 3360
   ccttcttgac gagttcttct gagcgggact ctggggttcg aaatgaccga ccaagcgacg 3420
   cccaacctgc catcacgaga tttcgattcc accgccgcct tctatgaaag gttgggcttc 3480
   ggaatcgttt tccgggacgc cggctggatg atcctccagc gcggggatct catgctggag 3540
   ttcttcgccc accctagggg gaggctaact gaaacacgga aggagacaat accggaagga 3600
   acccgcgcta tgacggcaat aaaaagacag aataaaacgc acggtgttgg gtcgtttgtt 3660
   cataaacgcg gggttcggtc ccagggctgg cactctgtcg ataccccacc gagaccccat 3720
   tggggccaat acgcccgcgt ttcttccttt tccccacccc accccccaag ttcgggtgaa 3780
   ggcccagggc tcgcagccaa cgtcggggcg gcaggccctg ccatagcctc aggttactca 3840
   tatatacttt agattgattt aaaacttcat ttttaattta aaaggatcta ggtgaagatc 3900
   ctttttgata atctcatgac caaaatccct taacgtgagt tttcgttcca ctgagcgtca 3960
   gaccccgtag aaaagatcaa aggatcttct tgagatcctt tttttctgcg cgtaatctgc 4020
   tgcttgcaaa caaaaaaacc accgctacca gcggtggttt gtttgccgga tcaagagcta 4080
   ccaactcttt ttccgaaggt aactggcttc agcagagcgc agataccaaa tactgtcctt 4140
   ctagtgtagc cgtagttagg ccaccacttc aagaactctg tagcaccgcc tacatacctc 4200
   gctctgctaa tcctgttacc agtggctgct gccagtggcg ataagtcgtg tcttaccggg 4260
   ttggactcaa gacgatagtt accggataag gcgcagcggt cgggctgaac ggggggttcg 4320
   tgcacacagc ccagcttgga gcgaacgacc tacaccgaac tgagatacct acagcgtgag 4380
   ctatgagaaa gcgccacgct tcccgaaggg agaaaggcgg acaggtatcc ggtaagcggc 4440
   agggtcggaa caggagagcg cacgagggag cttccagggg gaaacgcctg gtatctttat 4500
   agtcctgtcg ggtttcgcca cctctgactt gagcgtcgat ttttgtgatg ctcgtcaggg 4560
   gggcggagcc tatggaaaaa cgccagcaac gcggcctttt tacggttcct ggccttttgc 4620
   tggccttttg ctcacatgtt ctttcctgcg ttatcccctg attctgtgga taaccgtatt 4680
   accgccatgc at                                                     4692
<210> 17
   <211> 45
   <212> DNA
   <213> Künstliche Sequenz
<220>
   <223> Beschreibung der künstlichen Sequenz: primer
<400> 17
   gagagctagc agactcgagc tcttacatac atgtacttat aaaac 45
<210> 18
   <211> 34
   <212> DNA
   <213> Künstliche Sequenz
<220>
   <223> Beschreibung der künstlichen Sequenz: primer
<400> 18
   gagaggtacc agttaaagtt aatccttctg agag 34
<210> 19
   <211> 33
   <212> DNA
   <213> Künstliche Sequenz
<220>
   <223> Beschreibung der künstlichen Sequenz: primer
<400> 19
   gagagcggcc gcctcatact cgagggaaat tcg 33
<210> 20
   <211> 33
   <212> DNA
   <213> Künstliche Sequenz
<220>
   <223> Beschreibung der künstlichen Sequenz: primer
<400> 20
   gagaggatcc ggtaatctgc gtcttgccat cag 33
<210> 21
   <211> 12
   <212> DNA
   <213> Künstliche Sequenz
<220>
   <223> Beschreibung der künstlichen Sequenz: primer
<400> 21
   cggctggttc ta 12
<210> 22
   <211> 22
   <212> DNA
   <213> Künstliche Sequenz
<220>
   <223> Beschreibung der künstlichen Sequenz: primer
<400> 22
   gtcgactacg tcgttaaggc cg 22
<210> 23
   <211> 23
   <212> DNA
   <213> Künstliche Sequenz
<220>
   <223> Beschreibung der künstlichen Sequenz: primer
<400> 23
   acaaagctcc tctcctgctc aag 23
<210> 24
   <211> 22
   <212> DNA
   <213> Künstliche Sequenz
<220>
   <223> Beschreibung der künstlichen Sequenz: primer
<400> 24
   actaggatga gtagcagcac gt   22

## Beschreibung

Die Erfindung betrifft modifizierte Hefestämme sowie Verfahren zur Konstruktion dieser Hefestämme und deren Verwendung zur Prüfung der Genound/oder Zytotoxizität komplexer Umweltkontaminationen. Im einzelnen werden Hefestämme, insbesondere *Saccharomyces cerevisiae* Stämme offenbart, in denen die Nukleinsäuresequenzen für Rezeptor- und Reportersignalpotenzen für geno- und zytotoxische Umweltkontaminationen, wie z.B. das "green-fluorescent" Gen aus *Aequoria victoria* und das "red fluorescent" Gens aus der indopazifischen See-Anemonenspezies *Discosoma,* stabil in das Hefegenom integriert sind. Diese Hefestämme sind als biologische Komponente eines Biosensors einsetzbar, der zur dosisabhängigen geno- und zytotoxischen Substanzprüfung auf verschiedene und insbesondere zinnorganischer Umweltgifte, d.h. die Detektion aller in der Messprobe vorkommenden Schadstoffe, inklusive möglicher toxischer Abbauprodukte geeignet ist.

### Hintergrund der Erfindung

Wissenschaftliche Untersuchungen an niederen Tieren ergaben eine extreme Störung des hormonellen und des morphologischen Reproduktionssystems durch den Einfluss von Organozinnverbindungen, die zu Unfruchtbarkeit bis zum Aussterben bestimmter Arten geführt hat. Da die Synthese der Sexualhormone im gesamten Tierreich und auch beim Menschen nach den selben Prinzipien verläuft, kann eine negative Wirkung auf das hormonelle Reproduktionssystem des Menschen nicht ausgeschlossen werden. Bei Säugetieren beeinflusst TBT (Tri-butyl-tin) das Hormongleichgewicht durch Wirkungen auf endokrine Drüsen wie die Hypophyse, die Schilddrüse und die Hormondrüsen der Gonaden. Das Cytochrom-P450 abhängige Aromatase System spielt bei der Umwandlung von männlichen (Androgene), die im weiblichen Geschlecht stets die Vorstufe der weiblichen Geschlechtshormone (Östrogene) sind, eine bedeutende Rolle. In verschieden Forschungsstudien wurde nachgewiesen, daß TBT auf der Stufe der Cytochrom-P450 abhängigen Aromatase in den endogenen Steroidstoffwechsel mariner Gastropoden eingreift und die Aromatisierung von Androgenen zu Östrogenen hemmt, wie beschrieben in Bettin, R. et al., Phys. Rev. Stat. Phys. Plasmas Fluids Reiat. Interdiscip. Topics 51:212-219 (1995).

Dasselbe Enzymsystem, das auch als multifunktionelles Oxygenasesystem (MFO-System) bezeichnet wird und bei Mollusken sowie bei Säugern und beim Menschen nachgewiesen wurde, katalysiert sowohl die Aromatisierung zu Östrogenen als auch den Abbau von TBT. Der durch die - wahrscheinlich kompetitive - Hemmung der Cytochrom-P450 abhängigen Aromatase resultierende erhöhte Androgengehalt induziert bei weiblichen Tieren die zusätzliche Ausbildung der sekundären männlichen Geschlechtsmerkmale. Aufgrund dieser Ergebnisse und der Tatsache, daß die Steroidbiosynthese im gesamten Tierreich nach denselben Prinzipien abläuft, kann eine negative Wirkung von TBT und anderen Organozinnverbindungen auf das Cytochrom-P450 abhängige Aromatase-System höher entwickelter Lebewesen nicht ausgeschlossen werden. Möglicherweise kann die Umweltbelastung mit TBT und anderen Organozinnverbindungen als ein Faktor angesehen werden, der für die kontinuierlich zunehmenden Reproduktionsstörungen im weiblichen Geschlecht sowohl beim Menschen wie auch bei marin- oder limnischaquatisch lebenden Tieren verantwortlich ist. In einer vom World Wildlife Foundation, USA, veröffentlichten Studie werden Reproduktionsstörungen sowohl im männlichen als auch im weiblichen Geschlecht bei über 26 Tierarten aus aquatischen Biotopen beschrieben. Es liegen keine systematischen quantitativen und qualitativen Studien zur Akkumulation und Toxizität zinnorganischer Verbindungen im Menschen vor. Alarmierend sind allerdings die *in vitro* Untersuchungen der Arbeitsgruppe Endokrinologie am Institut für Klinische Biochemie in Bonn, wonach das zur Umwandlung von Androgenen in Östrogene notwendige Enzym Aromatase aus Uterusendothelzellen bereits durch Nanogramm-Mengen komplett inhibiert wird (D. Klingmüller, Institut für Klinische Biochemie, Universität Bonn, persönliche Mitteilung).

Gravierende Effekte von umweltrelevanten Chemikalien auf den Hormonhaushalt und auf die Fertilität im männlichen und weiblichen Geschlecht wurden z.B. für DDE (2,2-Bis(4-chlorophenyl)-1,1-dichlorethan; 4,4' DDE), ein Abbauprodukt des landwirtschaftlich verwendeten Pestizids Keltane durch die Entwicklung von Hermaphroditen bei männlichen Alligatoren in einem Seengebiet Floridas, USA beschrieben.

In England ist bei Fischen die Induktion von Sterilität im männlichen Geschlecht durch Nonylphenole nachgewiesen worden. Nonylphenole, deren Jahresproduktion allein in Großbritannien 20.000 Tonnen betragen, werden bei der Kunststoffherstellung eingesetzt und gelangen bei der Produktion und beim Gebrauch von Kunststoffen zunächst in die Abwässer und danach in Oberflächengewässer wie Bäche, Flüsse und Seen. Die Intoxinierung von Menschen mit TMT (Tri-Methy-Tin), einer potenten neurotoxischen Organozinnverbindung, resultierte in Epilepsie, Amnesie und Hippocamopusschädigungen, wie beschrieben in Feldmann, R. G. et al. Arch. Neurol. 50:1320-1324 (1993); Kreyberg, S. et al., Clin. Neuropathol. 11:256-259 (1992).

Für TMT und das verwandte TBT wurden ebenso selektiv Hippocampusregionen betreffende neurotoxische Effekte gezeigt, wie beschrieben in Walsh, T. J. et al., Neurobehav. Toxicol. Teratol 4:177-183 (1982); Balaban, C. D. et al., Neuroscience, 28:337-361 (1988) und Tsunashima, K. et al., Synapse 29:333-342 (1998).

Das Biozid Tributylzinn (TBT) gehört nach Auffassung der World Health Organisation (WHO) zu den giftigsten Stoffen, die je hergestellt und in die Umwelt entlassen worden sind. Die Toxizität und Wirksamkeit von TBT ist nur vergleichbar mit der des Dioxin. TBT wird meist als Oxid, Fluorid, Sulfid, Chlorid oder Acetat produziert und dissoziiert in wässrigen Lösungen unter Bildung eines hydratisierenden Kations, das sich durch hohe Bioverfügbarkeit auszeichnet.

Organozinnverbindungen werden seit Anfang der 50er Jahre, nach Entdeckung ihrer bioziden Wirkung, in der Industrie zur Imprägnierung, Stabilisierung und Konservierung verschiedenster Produkte eingesetzt. Die Hauptanwendungsgebiete von TBT und Triphenylzinn (TPhT) sind der Einsatz in konventionellen Antifoulingfarben mit Kontaktauslaugung, in ablativen Antifoulingfarben und in selbstschleifenden Copolymeren. Organozinnverbindungen werden in großen Mengen als Thermo- und/oder UV-Stabilisatoren bei fast allen PVC-Verarbeitungsverfahren eingesetzt (Kalandrier- und Extrusionsverfahren, Blasformen und Spritzgussverfahren). Dieser Verwendungsbereich ist der mengenmäßig bei weitem bedeutendste, bezogen auf alle Anwendungsbereiche zinnorganischer Verbindungen. Weltweit werden etwa 75.000 t/a an Organozinnstabilisatoren eingesetzt; in Europa liegt der Verbrauch bei ca. 15.000 t/a, in Deutschland bei etwa 5.000 t/a (Verbrauchszahlen jeweils für das Jahr 1999). Aufgrund ihrer bioziden Wirkung werden diese Chemikalien auch als Holz- und Materialschutzmittel für Textilien, Dichtungs- und Vergussmassen (z. B. Polyurethanschäume), für Anstriche und Klebstoffe sowie mineralische Materialien (z. B. Dämmstoffe) und als Kunststoffstabilisatoren eingesetzt. Organozinnverbindungen werden in der Landwirtschaft, im Gartenbau und in der Tierhaltung als Biozide gegen Pilze, Bakterien, Ameisen, Milben, Nematoden, Insekten, Mollusken und Nagetiere eingesetzt. Außerdem spielt die Anwendung in der Papier- und Brauereiwirtschaft, an Kühltürmen, bei der Lederimprägnierung, in Dispersionsfarben und als Desinfektionsmittel eine Rolle.

Der Mensch kommt durch die vielfältige Anwendung von Organozinnverbindungen auf verschiedene Weise mit diesen Verbindungen in Kontakt. Auswascheffekte führen
i) zu einer enormen Belastung sowohl küstennaher mariner als auch limnischer Gewässer und Sedimente und damit zur Belastung mariner Lebensmittel (nahrungsbedingte orale Aufnahme) wie beschrieben in Nilsson R. Toxicol Pathol. 28:420-31 (2000);
ii) zu Einträgen in das Süß- und Sickerwasser und einer kontinuierlichen Belastung der Abwässer. Emissionen von TBT aus imprägnierten Hölzern sowie der Einsatz als Biozide zum Schutz von Textilien, Tapeten, Wandfarbe, Papier, mineralischen Dämmstoffen, Silikon und Polyurethanschäumen führen zu Belastungen der Innenraumluft (inhalative Aufnahme über die Atmung). Direkte Kontaktmöglichkeiten zur Aufnahme über die Haut (percutan) stellen Textilien dar, die zum Schutz des Gewebes mit TBT imprägniert werden.

Die Kontrolle der Abwassereinleitung vor ökotoxischer Belastung erfolgt neben der chemischen Vollanalyse über standardisierte Biotests (DIN, ISO) mit Bakterien, Algen, Kleinkrebsen und Fischen als Indikatororganismen. Organozinnverbindungen werden bisher ausschließlich qualitativ mit gas- oder flüssig-chromatographischen Verfahren nachgewiesen, wie beschrieben in Chiron S. et al., J Chromatogr A. 879:137-45 (2000); Gonzalez-Toledo E. et al., J. Chromatogr A. 878:69-76 (2000); Millan, E., Pawliszyn, J. Chromatogr A. 873:63-71 (2000).

Diese Verfahren sind technisch aufwendig und teuer, der einzige deutsche Anbieter entsprechender Analytik ist die Firma Galab in Geesthacht. Auf der ACHEMA Messe (Juli 2000, Frankfurt) wurde von dem ICB Institut (Münster) in Zusammenarbeit mit der Firma Gerstel der Prototyp eines Gaschromatographen vorgestellt, der zinnorganische Verbindungen nachweisen kann, das einzelne Gerät ist mit 100.000 DM zu veranschlagen und ab etwa Frühjahr 2001 im Handel. Die Empfindlichkeit moderner chemisch-analytischer Methoden für allgemeine Umwelttoxine inklusive der biochemischen (Enzymund Immunoassays) liegen weit unter der ökotoxischen Wirkungsschwelle.

Bioassays zur Analyse komplexer geno- und zytotoxischer Noxen inklusive zinnorganischer Verbindungen sind bisher nicht im Einsatz. Zur Detektion von allgemeinen Umweltgenotoxinen werden u. a. zahlreiche prokaryotische Testsysteme eingesetzt. Beispiele sind der AMES-Test (Ames, B.N. et al., Proc. Natl. Acad. Sci. USA 70, 2281-2285 (1973)) und der bakterielle SOS-Lux-Test (Ptitsyn, L. R. et al., Applied and Environmental Microbiology 63: 4377-4384 (1997) und Horneck, G. et al., Biosensors for Environmental Diagnostics, Teubner, Stuttgart, pp. 215-232 (1998)). Im prokaryotischen Lux-Fluoro-Test werden rekombinante *Salmonella typhimurium* TA1535 Bakterien-Zellen eingesetzt (Rettberg, P. et al., Analytica Chimica Acta 387: 289-296 (1999)). Ein anderes, in der WO 99/53092 beschriebenes prokaryontisches Testsystem verwendet zur Feststellung der spezifischen Genotoxizität im Vergleich zur potentiell gleichzeitig auftretenden Zytotoxizität zwei verschiedene Mikroorganismen, wobei einer mit einem Plasmid transformiert ist, das einen Stressinduzierbaren Promotor und eine Reportergen (Lux-Gen) trägt. Der andere Stamm ist mit einem Plasmid transformiert, dass einen konstitutiven Promotor und ein Reportergen (Lux-Gen) enthält. Durch Vergleich der Reaktionen der beiden Mikroorganismen auf eine Probe kann auf ihre Genound Zytotoxizität geschlossen werden.

Die vorhandenen prokaryotischen Biotests zur Detektion von Umwelttoxinen haben die Nachteile, dass
(i) zeitverzögerte Reaktionen mit dem Absterben der Organismen/Zellen gemessen werden,
(ii) keine Erfassung all jener Substanzen, die über ihre Interaktionen mit Zellstrukturen in höheren Organismen Mutationen auslösen (z. B. durch Störungen des Spindelapparates)
(iii) keine genotypisch stabilen differenzierten Rezeptor- und Reporterkomponenten für genotoxische und/oder zytotoxische Agentien enthalten sind, und
(iv) aktive bakterielle Effluxsysteme für hydrophobe Substanzen nicht ausgeschaltet sind (siehe auch van Ween, H.W. & Konings W.N. Biol. Chem. 378: 769-777 (1997) und Cloeckert, A. & Schwarz, S. Vet. Res. 32:301-310 (2001) und Daly, M. & Fanning, S. Appl. Environ. Microbiol. 66:4842-4848 (2000)).

Ein eukaryontisches Testsystem basierend auf *Saccharomyces cerevisiae* Zellen wurde von Billington et al. (Biosensors & Bioelectronics 13, 831-838 (1998)) beschrieben. In diesem System werden integrative und replizierende Reporterkonstrukte offenbart, in denen eine mit dem RAD 54-Promotor gekoppelte Gensequenz für ein grün fluoreszierendes Protein (yEGFP), durch DNA-schädigende Substanzen induzierbar ist. Dieses System kann zur Detektion von genotoxischen Substanzen eingesetzt werden. Eine Vorläuferpublikation zu diesem Thema ist die von Walsmley et al., Yeast 13: 1535-1545 (1997) in der die Integration und autonome Republikation solcher Reporterkonstrukte beschrieben ist.

Ein kürzlich publizierter Biotest zur Bestimmung aquatischer Toxizität beruht auf der Gärleistung von Saccharomyces cerevisiae Zellen (Weber, J. et al., Z. Umweltchem. Ökotox. 12:185-189 (2000) und Weber et al. Vom Wasser 95:97-106 (2000)). Dieser Vorgang kann jedoch nicht automatisiert vorgenommen werden und dauert etwa 25 Stunden.

Außerdem exprimieren Wildtyp Zellen der Hefe *Saccharomyces cerevisiae* eine durch die ABC (ATP-binding-cassette = ATP-Bindungs-Kassette)-Transporter Gene *PDR5, SNQ2* und *YOR1* vermittelte erhebliche endogene Resistenz gegenüber zinnorganischen Verbindungen sowie einer Vielzahl hydrophober aber auch metallhaltiger Substanzen (Golin, J. et al., Antimicrob. Agents Chemother. 44:134-138 (2000)). Damit sind *Saccharomyces cerevisiae* Wildtyp Stämme für umweltbiotechnologische Zwecke (die Detektion umweltrelevanter Noxen) nicht geeignet. Die Nachteile zur Verwendung der Wildtyp Stämme sind insbesondere:
(A) kein definierter genetischer Hintergrund,
(B) metabolische Messparameter unterliegen einer komplexen metabolischen Regulation und zeigen somit nur ungenau mögliche Intoxinierungen, da
(C) die Expression der für die endogene Resistenz verantwortlichen ABC-Transporter die Zellen und damit metabolische Funktionen schützt, und
(D) somit die Auswertung der erhaltenen Daten in Bezug auf toxikologische Effekte wässriger Lösungen und Substanzen erschwert ist.

Die intensive Verwendung zinnorganischer Verbindungen in einer Reihe industrieller Prozesse mit zunehmender Freisetzung und Akkumulation in der Umwelt ist gegenwärtig Gegenstand erheblicher umweltpolitischer und öffentlicher Besorgnis

Andererseits besteht jedoch seitens der Industrie ein Bedarf an der Verwendung dieser oder ähnlicher Substanzen im Produktionsprozess.

Somit besteht ein dringender Bedarf eines Nachweisverfahrens zur schnellen Erfassung allgemein umweltrelevanter Noxen wie z.B. Organozinnverbindungen, ionisierende und nicht-ionisierende Strahlung (UV-Strahlung) aber auch chemischer Karzinogene. Die hohe Homologie essentieller zellulärer Vorgänge in Hefe und Zellen höherer Eukaryoten lässt den Schluss zu, dass diese als eukaryotisches Detektions- und Analysesystem zur Identifizierung geno- und zytotoxischer Verbindungen allgemeiner Noxen geeignet sein könnte. Solche Hefestämme könnten in Testreihen für Durchmusterungen möglicher kontaminierter Lösungen in sehr kleinem Maßstab mit hohem Wirkungsgrad (Bioassay) eingesetzt werden.

### Zusammenfassung der Erfindung

Die Aufgabe und das Ziel der vorliegenden Erfindung war es, die o.g. Nachteile zu vermeiden und ein eukaryotisches, auf Hefezellen basierendes Testsystem zur dosisabhängigen geno- und zytotoxischen Substanzprüfung umweltrelevanter Noxen und insbesondere zinnorganischer Umweltgifte zu entwickeln. Es wurde nun gefunden, dass ein genetisch definierter (isogener) Hefewirtsstamm, z. B. ein *Saccharomyces cerevisiae* Hefewirtsstamm, in dem geno- und zytotoxische Signalpotenz stabil in das Hefegenom integriert ist und exprimiert wird, ein geeignetes Testsystem ist. Zusätzlich können in dem Hefewirtsstamm Xenobiotikatranslokationsgene, die für die endogene Resistenz verantwortliche ABC-Transportergene kodieren, spezifisch deletiert sein. Dieses Testsystem ermöglicht die Detektion aller in der Messprobe vorkommenden Schadstoffe, inklusive möglicher toxischer Abbauprodukte.

Die vorliegende Erfindung betrifft somit
(1) einen modifizierter Hefestamm in dem
   (a) eine Genotox-Kassette, umfassend einen ersten Promoter und ein erstes Reportergen, das funktional mit dem ersten Promoter verknüpft ist, und
   (b) eine Zytotox-Kasette, umfassend einen zweiten Promoter und ein zweites Reportergen, das funktional mit dem zweiten Promoter verknüpft ist,
   wobei die Promoter und Reportergene in (1) und (2) voneinander verschieden sind, stabil und funktional in das Genom eines Hefewirtsstammes integriert sind;
(2) eine bevorzugte Ausführungsform von (1), wobei der Hefestamm durch Disruption oder Deletion einer oder mehrerer der in dem Hefewirtsstamm vorhandenen Xenobiotikatranslokationsgene sensibilisiert ist;
(3) ein Verfahren zur Herstellung eines modifizierten Hefestamms, wie vorstehend in (1) oder (2) definiert, umfassend das Integrieren einer Genotoxund einer Zytotox-Kassette in den Hefewirtsstamm;
(4) ein Verfahren zur Detektion umweltrelevanter Noxen, umfassend:
   (a) die Behandlung eines modifizierten Hefestammes, wie vorstehend in (1) oder (2) definiert, mit einer Testsubstanz oder einem Testsubstanzgemisch;
   (b) Wachstumsbestimmungen in Anwesenheit oder nach Beendigung der Behandlung mit der Testsubstanz/dem Testsubstanzgemisch, und
   (c) Messungen des Anstiegs oder der Abnahme der Reportergenaktivität des Hefestammes in Anwesenheit oder nach Beendigung der Behandlung mit der Testsubstanz/dem Testsubstanzgemisch;
(5) Verwendung eines modifizierten Hefestamms, wie vorstehend in (1) oder (2) definiert, zur Prüfung der Geno- und/oder Zytotoxizität komplexer Umweltkontaminationen; und
(6) Testkit und Biosensor zur Prüfung der Geno- und/oder Zytotoxizität komplexer Umweltkontaminationen, umfassend einen modifizierten Hefestamm, wie vorstehend in (1) oder (2) definiert.

Die erfindungsgemäßen modifizierten Hefewirtsstämme sind insbesondere zur dosisabhängigen geno- und zytotoxischen Substanzprüfung verschiedener und insbesondere zinnorganischer Umweltgifte, d.h. die Detektion aller in der Messprobe vorkommenden Schadstoffe, inklusive möglicher toxischer Abbauprodukte, geeignet. Diese Hefezellen - mit stabil in das Genom integrierter Rezeptor- und Reportersignalpotenzen - sind somit in Testkits und Biosensoren für geno- und/oder zytotoxische Umweltkontaminationen einsetzbar. Die Zellstruktur (Plasmamembran, intrazelluläre Membransysteme, Zellorganellen, Enzymausstattung) der Hefe gleicht prinzipiell den Zellen höherer Organismen. Wegen der leichteren Kultivierbarkeit (Verdopplungszeit von etwa 90 Minuten) sind Hefezellen jedoch ungleich leichter zu handhaben als etwa Gewebszellen von Säugetieren. Die konstruierten Hefestämme stellen die Basis eines biotechnologischen Analyse- und Durchmusterungssystems dar.

### Figurenbeschreibung

Fig. 1: Vektor pUC18pma1.
Fig. 2: Vektor p774.
Fig. 3: Genomisch integrierte Signalpotenz zur Zytotoxizitätsprüfung.
Fig. 4: Genomisch integrierte Signalpotenz zur Genotoxizitätsprüfung.
Fig. 5: Überprüfung der Integration von
   (A) pma1-DsRed1 am Leu2-Locus (Oligonukleotide: leu2int_antisensel/pmal-158_antisense; Positiv: Hefeklone Nr. 2, 3, 4) und
   (B) egfp-URA3 am RAD54-Locus (Oligonukleotide: prerad_sense_intl/ura3_antisense; Positiv: Hefeklon Nr. 3).
Fig. 6: Fotografien des erfindungsgemäßen *S.-cerevisiae*-Hefestamms in Fluoreszenztests von Beispiel 3.3 nach 8 Stunden Inkubation mit (A) 0,05 ng/ml, (B) 0,5 ng/ml Mitomycin C und (C) 0,1 ng/ml, (D) 0,01 ng/ml TPhT.
Fig. 7: Wachstum des erfindungsgemäßen *S.-cerevisiae*-Hefestamms nach 8 Stunden Inkubation in Abhängigkeit von der Inhibitorkonzentration im Kulturmedium.

### Detaillierte Beschreibung der Erfindung

"Funktional" und "funktional verknüpft" im Sinne der vorliegenden Erfindung bedeutet, dass die entsprechenden Gene so angeordnet sind bzw. so in das Genom der Hefewirtsstämme integriert sind, dass sie - in Abhängigkeit von dem "Schaltzustand" des Promoters - exprimiert werden.

"Stabil integriert" im Sinne der vorliegenden Erfindung bedeutet, dass bei der mitotischen Vermehrung der Hefestämme das entsprechende Merkmal ohne externen Selektionsdruck immer erhalten bleibt und an die Nachkommenschaft weitergegeben wird.

Der modifizierte Hefestamm gemäß Ausführungsformen (1) und (2) der Erfindung ist insbesondere ein Hefestamm der Ordnung *Ascomycota,* besonders bevorzugt ein Hefestamm der Gattungen *Saccaromycetales, Candidaceae* oder *Kluyveromyces*. Hiervon sind die Hefen der Gattung *Saccharomycetales,* insbesondere die der Familie *Saccharomycetaceae,* besonders bevorzugt. Geeignete Saccharomycetaceae sind die Spezie *Saccharomyces cerevisiae* und *Saccharomyces uvarum*, wobei *S*. *cerevisiae* bevorzugt ist.

"Verschiedene Reportergene" bedeutet, dass die beiden exprimierten Reporter bei gemeinsamer Expression in dem modifizierten Hefestamm identifizierbar und quantifizierbar sind.

"Verschiedene Promotoren" im Sinne der vorliegenden Erfindung bedeutet, dass die in der Genotox- und in der Zytotox-Kasette eingesetzten Promotoren unabhängig voneinander durch genotoxische bzw. zytotoxische Agentien induzierbar sind und die Expression der jeweiligen mit ihnen funktionell verknüpften Reportergene ermöglichen.

Der in der Genotox-Kassette vorhandene erste Promoter ist vorzugsweise ein Promoter der durch genotoxische Agentien induziert wird und Reparaturmechanismen ansteuert, die als Folge von primären DNS-Schäden aktiviert werden. Dazu zählen sowohl heterologe Promotoren, wie z. B. der prokaryotische SOS Promotor (Oda, Y., Mutat. Res. 147:219-229 (1985) und Reiferscheid, G., et al., Mutat. Res. 253:215-223 (1991), als auch homologe Promotoren zur Regulation von Gen- oder Zellreparaturgenen. In der vorliegenden Erfindung wird in der Gentox-Kasette vorzugsweise ein homologer Promoter, insbesondere ein Promoter der RAD-Gene (wie RAD54, RAD26 und RDH454, wobei die in SEQ ID NOs: 1 bis 3 gezeigten RAD54-, RAD26- und RDH54-Promoter besonders bevorzugt sind) oder der Heat-Schock-Gene (wie HSP70 und HSP82, wobei die in SEQ ID NOs:4 und 5 gezeigten HSP70- und HSP82-Promoter besonders bevorzugt sind) eingesetzt.

Der in der Zytotox-Kassette vorhandene zweite Promoter ist vorzugsweise ein Promoter, der die konstitutive Expression von Haushaltsgenen reguliert und durch zytotoxische Agenzien deaktiviert wird. Es sind sowohl heterologe als auch homologe Promotoren einsetzbar. In der vorliegenden Erfindung wird in der Zytotox-Kasette vorzugsweise ein homologer Promoter, insbesondere ein Promoter eines Tubulins (wie α-Tubulin-Promoter einschließlich TUB1- und TUB3-Promoter, wobei die in SEQ ID NOs:6 und 7 gezeigten TUB1 und TUB3-Promoter besonders bevorzugt sind) oder eines Stoffwechselenzyms (wie PMA1-, PMA2- und H⁺-ATP-ase-Promoter, wobei die in SEQ ID NOs:8 und 9 gezeigten PMA1- und PMA2-Promoter besonders bevorzugt sind) eingesetzt.

Das erste und zweite Reportergen kann jedes beliebige Reportergen sein, vorausgesetzt, dass die beiden Reportergene nicht interferieren, d. h. getrennt nachgewiesen werden können. Geeignete Reportergene sind z. B. Fluoreszenzmarker (z. B. das Green Fluorescent Protein (GFP) aus Aequorea victoria, Red Fluorescent Protein, wie aus der indopazifischen Seeanemonenspezies *Discosoma* bzw. für die Verwendung in Hefen adaptierte Mutanten hiervon), Enzyme (insbesondere solche, die von der Hefe sezerniert und dann mittels Farbreaktion nachgewiesen werden können wie Peroxidasen, Esterasen und Phosphorylasen) oder Antigene (die durch Immunoassays nachgewiesen werden können wie c-myc und Hab). Besonders bevorzugt ist die Verwendung von zwei nicht interferierende Fluoreszenzmarkern.

In einer besonders bevorzugten Ausführungsform umfassen die beiden Reportergene Nukleinsäuresequenzen, die für das "green-fluorescent" Gen aus *Aequorea victoria* oder Mutanten hiervon sowie für das "red fluorescent" Gen aus der indopazifischen Seeanemonenspezies *Discosoma* oder eine Mutante hiervon kodieren. Besonders bevorzugt sind die Mutanten der fluoreszierenden Proteine, die von den in SEQ ID NO:10 und 12 gezeigten DNA-Sequenzen kodiert sind. Die Genotox- und Zytotox-Kassetten können weitere funktionelle DNA-Sequenzen/Gene aufweisen wie z. B. Selektions-/-Markergene (nachfolgend auch "selektierbare Marker"), die u. a. zur Selektion auf erfolgte Integration dienen können, sowie Rekombinase-Erkennungssequenzen und Spleißstellen, die zum Entfernen von unerwünschten Abschnitten in der eingeführten Kassette wie z. B. der Selektions-/-Markergene dienen.

Die selektierbaren Marker können dabei sowohl Auxotrophie-Marker wie die Auxotrophiemarker URA3 (siehe SEQ ID NO:14) und LEU2 oder Gene, die eine Resistenz z.B. gegen G418 (Aminoglycosid-Phosphotransferase-Gen) bewirken.

Gemäß der Ausführungsform (2) des Hefestammes sind ein oder mehrere der in dem Hefewirtsstamm vorhandenen Xenobiotikatranslokationsgene, die zur Ausschleusung toxischer Substanzen notwendig sind, deletiert bzw. disrupiert. Solche Translokationsgene (auch "ABC-Transportergene" genannt) sind in den nachfolgenden Tabellen 1 und 2 zusammengefasst.

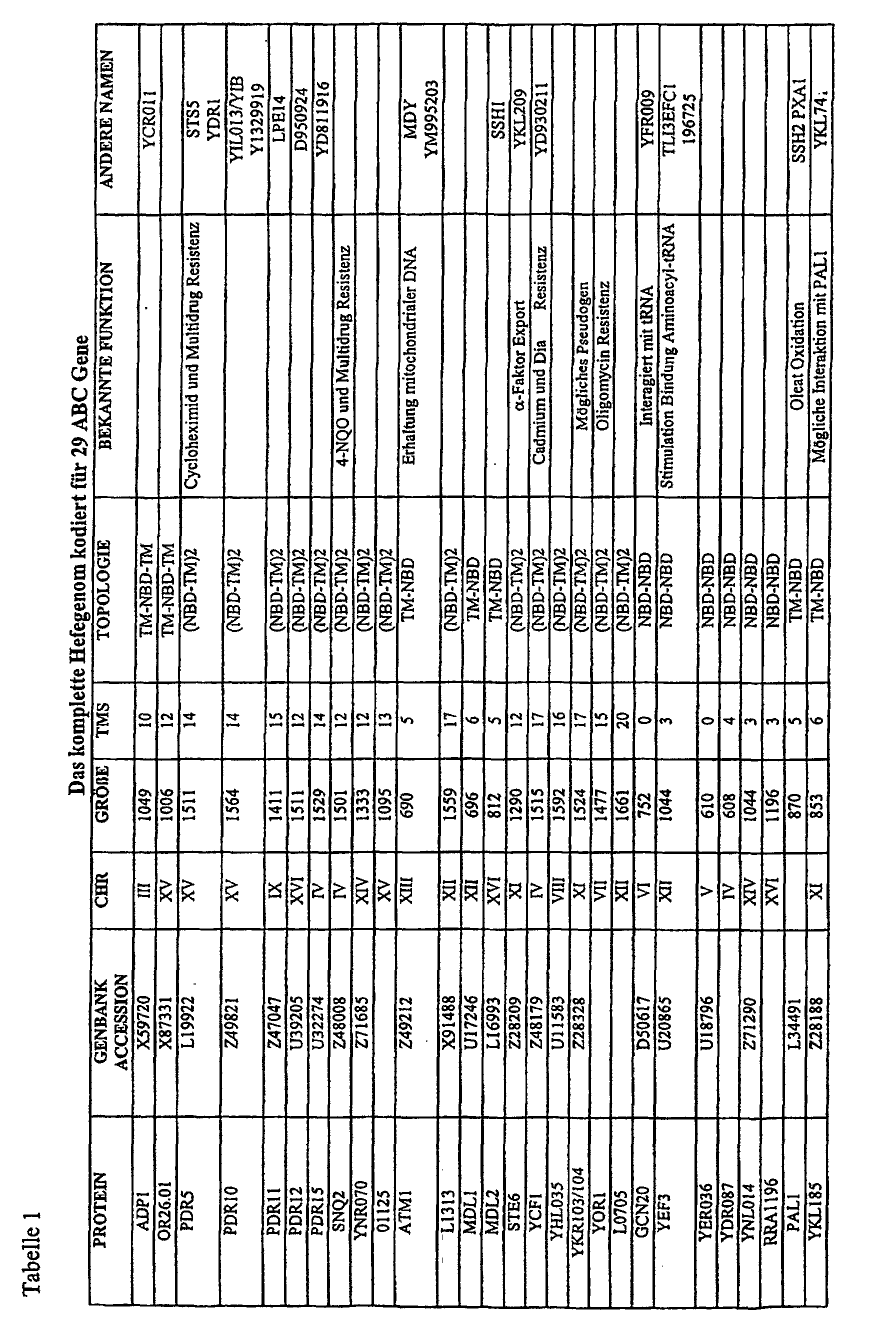

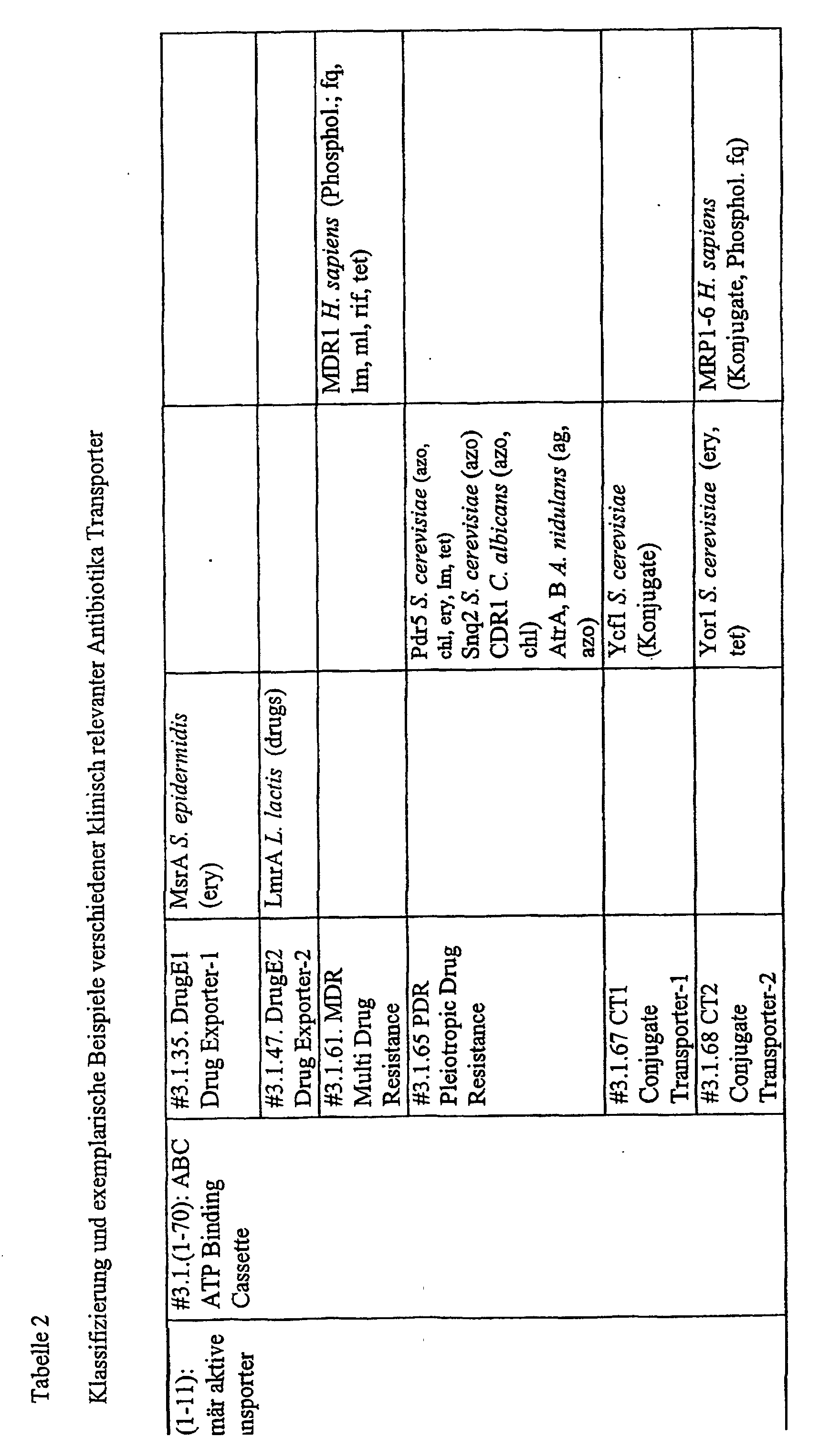

Bei den Xenobiotikatranslokationsgenen, die deletiert bzw. disrupiert werden, handelt es sich u. A. um PDR5, YOR1, SNQ2, YCF1, PDR10, PDR11 und PDR12. Bevorzugt ist dabei, dass wenigstens das PDRS-Gen deletiert ist, besonders bevorzugt ist ein modifizierter, isogener *Saccharomyces-cerevisiae*-Hefewirtsstamm mit Deletionen in den *PDR5-*, *YOR1-* und *SNQ2*-Genen.

Das Verfahren gemäß Ausführungsform (3) der Erfindung umfasst das Einführen der Kassette in das Hefegenom. Die Hefetransformation kann entsprechend der Lithium-Acetat Methode, wie von Rothstein, R. in Methods in Enzymology 194: 281-302 (1991) beschrieben, erfolgen. Hefegenetische Methoden, insbesondere für *Saccharomyces cerevisiae* erfolgen entsprechend der bei Sherman, F. et al., Cold Spring Harbor Laboratory, Cold Spring Harbor, New York (1981) beschriebenen Methode, die Kreuzung der modifizierten Stämme und Isolieren der diploiden Stämme durch Mikromanipulation umfasst. Die Integration wird dabei vorzugsweise mittels der vorstehend erwähnten selektiven Marker (Auxotrophie und/oder Resistenzen) verfolgt. Im einzelnen werden die Marker enthaltenden Kassetten eingeführt, die nachfolgende Kreuzung führt zum Erhalt isogener Stämme und Auswahl derjenigen Stämme, welche nach Transformation eine stabile Integration der gewünschten Kasetten in das Hefegenom aufweisen (z.B. im Falle des Plasmids *p774pma1Dsred* und der DNS Kassette *rad54::egfp* erfolg eine Anzucht in Kulturmedien und eine Auswahl der Stämme, die ohne die Zusätze von Leucin und Uracil wachsen).

Zum Erhalt des *pdr5yor1snq2* dreifach-mutanten Hefewirtsstammes, z. B. des *Saccharomyces-cerevisiae*-Hefewirtsstammes, können die Gene durch Einführung eines oder mehrerer selektierbarer Marker (Auxotrophie und/oder Resistenzen) deletiert und/oder disruptiert werden.

Die selektierbaren biosynthetischen Markergene (Auxotrophiebedürfnisse und/oder Resistenzen) können durch rekombinante DNS-Techniken in die Loci der Wildtyp-Kaliumtransportergene eingeführt werden. Geeignete selektierbare Marker sind vorstehend genannte Auxotrophie- und Resistenzmarker. Solche modifizierten Allele können dann in *Saccharomyces cerevisiae* transformiert werden, wo sie durch homologe Rekombination die Wildtyp-Loci ersetzen. Die Stämme mit modifizierten Allele können durch Selektion auf den oder die biosynthetischen Marker ermittelt werden.

Die in die Loci der unspezifischen Translokationssysteme eingeführten selektierbaren biosynthetischen Marker stellen außerdem einen einfachen Weg zum Transfer dieser Mutationen in genetisch andere Linien dar (Kreuzung). Ein Stamm, der eine Mutation in einem der Xenobiotikatranslokationsgene (z.B. PDR5 oder YOR1 oder SNQ2) beinhaltet, kann mit einem Stamm des entgegengesetzten Paarungstyps, der eine Mutation in einem anderen Xenobiotikatranslokationsgen (z.B. PDR5 oder YOR1 oder SNQ2) trägt, zu Diploiden gekreuzt werden. Durch anschließende Sporulation zu Haploiden (Tetradenanalyse) kann die isogene Nachkommenschaft dann auf die Anwesenheit der biosynthetischen Marker hin selektiert werden. Der *pdr5yor1snq2* dreifach-mutante *Saccharomyces-cerevisiae-*Phänotyp kann durch Wachstumstests auf selektiven Kulturmedien mit z. B. Ketokonazolkonzentrationen von 100 µM oder weniger ermittelt werden. Ein erfindungsgemäßer Hefestamm kann durch einen Test ermittelt werden, bei dem analysiert wird, ob eine Substanz den *pdr5yor1snq2* dreifach-mutanten *Saccharomyces-cerevisiae-*Phänotyp intoxiniert. Dazu wird der zu prüfende Stamm durch Wachstumstests auf selektiven Kulturmedien mit der Substanz inkubiert. Diese einfachen Verfahren, in denen Wachstumsveränderungen durch Agarplattentests und/oder in Flüssigkultur beobachtet werden kann, können somit spezifisch wirksame Substanzen detektieren, die metabolische Funktionen oder morphologische Veränderungen modulieren. Zur Prüfung verschiedener Substanzen oder Lösungen kann das Durchmusterungsverfahren solche Veränderungen wie metabolische Aktivität oder verminderte Wachstumsrate beinhalten. Die Testsubstanzen, die im Verfahren zur Detektion spezifischer Modulatoren eingesetzt werden, können z.B. synthetische oder natürliche Produkte sein. Natürliche Produkte beinhalten pflanzliche, tierische oder mikrobielle Extrakte.

Gemäß Ausführungsform (4) betrifft die vorliegende Erfindung ein Verfahren zur Detektion umweltrelevanter Noxen, das heißt eine Verfahren zur dosisabhängigen geno- und zytotoxischen Substanzprüfung, insbesondere auf zinnorganische Umweltgifte. Hierbei werden die erfindungsgemäßen Hefestämme in einer Nährlösung, vorzugsweise einer YNB-Nährlösung (1,7 g/l Hefestickstoffbasis ohne Aminosäuren), 5 g/l NH₄SO₄, 2 % D-Glucose, 0,5g/l Aminosäure-Mix (bestehend aus 250 mg Adenin, 500 mg Tryptophan, 100 mg Arginin, 100 mg Methionin, 150 mg Tyrosin, 150 mg Lysin, 300 mg Valin, 500 mg Threonin, 500 mg Serin, 250 mg Phenylalanin, 100 mg Asparagin, 10 mg Glutaminsäure, 100 mg Histidin)), bei pH 5,6 bis 5,9 bei 25 bis 35 °C, vorzugsweise 30 °C, 12 - 18 h unter Schütteln und Belüften vorinkubiert. Aliquots dieser vorinkubierten Lösung, die 1 x 10⁵ bis 1 x 10⁷ Hefezellen pro ml enthalten, werden mit bis zu 1 Vol.-% einer wässrigen Lösung der zu prüfenden Substanz bzw. bis zu 0,1 Gew.-% der zu prüfenden Substanz als Feststoff in Kontakt gebracht, unter den vorstehend beschriebenen Bedingungen der Vorinkubation bebrütet (ca. 5 - 20 h) und die Auswirkung auf die Reporterpotenzen in den bebrüteten Hefekulturen nachgewiesen. Falls erforderlich kann - bei positiven Befund - ein Abgleich mit einer Referenzprobe mit bekannter Noxenkonzentration erfolgen. Der Nachweis erfolgt spezifisch für die in dem modifzierten Hefestamm vorhandenen Reporter. Bei einem Reportersystem mit den vorstehend erwähnten "green" und "red fluorescent protein" erfolgt der Nachweis durch Messung der Fluoreszenzintensität bei 508 und 583 nm. Eine detaillierte Beschreibung für einen Test mit einem Hefestamm mit einem solchen Reportersystem ist in Beispiel 4 gegeben.

Weiterhin betrifft die vorliegende Erfindung einen Testkit und einen Biosensor (nachfolgend auch "Biotest"), umfassend die erfindungsgemäßen genetisch veränderten Hefezellen, insbesondere *Saccharomyces-cerevisiae*-Zel len. Dieser Biotest ist leicht zu handhaben und stellt eine wenig aufwendige Nachweismethode zur Erfassung geno- und zytoxischer Wirkungen komplexer Stoffgemische in wässrigen Lösungen bereit. Methodisch ist eine sterile Arbeitsweise nicht erforderlich. Der konstruierte hypersensitive, geno- und zytotoxisch signalgebende Hefestamm kann als biotechnologisches Hochdurchsatz-Testsystem zur konzentrationsabhängigen Detektion komplexer Umweltkontaminationen sowie insbesondere zinnorganischer Verbindungen in Lösungen eingesetzt werden.

Diese Technologie hat ein breites Einsatzspektrum zur Detektion umweltrelevanter Noxen in der Gesundheitsvorsorge für den Menschen als:
1. Frühwarnsystem in der Wasserüberwachung,
2. ökotoxikologische Bewertung von Abwässern,
3. Biotest in der Ökotoxikologie,
4. Funktionsüberwachung von Kläranlagen,
5. in der Medizin zum Toxizitätsscreening für Arzneimittel und Substanzen, und
6. in der Industrie zur Überwachung der im Produktionsprozess verwendeten Lösungen.

Der Vorteil des erfindungsgemäßen Testverfahrens besteht dabei
i) im Hinblick auf die Empfindlichkeit, mit der das aktive Material identifiziert werden kann,
ii) die Anzahl der Proben die getestet werden können sowie
iii) in der Schnelligkeit (8 Stunden), in der der Test durchgeführt werden kann,
iv) in der nicht-sterilen methodischen Durchführung des Tests, und
v) in der möglichen parallelen Detektion von geno- und zytotoxischen Effekten durch differenzierte Signale.

Die biotechnologische Verwendbarkeit stabil Geno- und Zytoxizität detektierender, gut wachsender Hefestämme besteht in der frühzeitigen Detektion gesundheitsgefährdender Umweltbelastungen sowie zur Abschätzung des Risikos für die Gesundheit des Menschen als: i) Frühwarnsystem in der Wasserüberwachung, ii) ökotoxikologische Bewertung von Abwässern, iii) Biotest in der Ökotoxikologie, iv) Funktionsüberwachung von Kläranlagen, v) in der Medizin zum Toxizitätsscreening für Arzneimittel und Substanzen, und vi) in der Industrie zur Überwachung der im Produktionsprozess verwendeten Lösungen, da Hefen in auf Wachstum basierenden Testreihen für Durchmusterungen vieler verschiedener Testlösungen in sehr kleinem Maßstab und mit hohem Wirkungsgrad ("Screening"-Verfahren in Mikrotiterschalen) verwendet werden können.

Schließlich betrifft die vorliegende Erfindung noch ein Verfahren zur Detektion spezifischer Modulatoren der exprimierten Reportergene, z.B. der pma1-Dsred1- und rad54::egfp-Reportergene, umfassend:
(a) die Behandlung eines Hefewirtsstamms, insbesondere eines *Saccharomyces cerevisiae* Hefewirtsstamms, in dem Rezeptor- und Reportersignalpotenzen für geno- und/oder zytotoxische Umweltkontaminationen wie vorstehend definiert stabil in das Genom integriert sind, aber nicht die der Hefe *Saccharomyces cerevisiae* eigenen PDR5, SNQ2 oder YOR1 Xenobiotikatranslokations-Systeme exprimiert sind, mit Testsubstanzen,
(b) Wachstumsbestimmungen in Anwesenheit oder nach Anwendung einer Testsubstanz, und
(c) Messungen des Anstiegs oder der Abnahme der Reportergenexpression, z.B. die Fluoreszenzintensität dieses Stammes in Anwesenheit oder nach Anwendung einer Testsubstanz.

Die nachfolgenden Beispiele erläutern die Erfindung weiter.

### Beispiele

### Allgemeine Methoden

Rekombinante DNA-Technik: Zur Anreicherung und Manipulation von DNS wurden Standardmethoden benutzt, wie sie bei Sambrook, J. et al., Cold Spring Harbor Laboratory Press, Cold Spring Harbor, New York (1989), beschrieben sind. Die verwendeten molekularbiologischen Reagenzien wurden nach den Angaben der Hersteller eingesetzt.

Hefetransformation: *Saccharomyces-cerevisiae*-Stämme wurden entsprechend der Lithium-Acetat Methode, wie von Rothstein, R. in Methods in Enzymology 194: 281-302 (1991), beschrieben, transformiert.

Hefegenetische Methoden: *Saccharomyces-cerevisiae*-Stämme wurden entsprechend der bei Sherman, F. et al., Cold Spring Harbor Laboratory, Cold Spring Harbor, New York (1981) beschriebenen Methode gekreuzt und diploide Stämme durch Mikromanipulation isoliert.

Primer: Genbank Accession SCYGL163C, 4837 bp, Chromosom VII, Leserahmen YGL163c, deponiert 11. August 1997:
Oligonukleotid postrad_sense (SEQ ID NO:17): Position 380 - 406 in der genomischen Sequenz, wobei die aus klonierungstechnischen Gründen angefügten Nukleotide kursiv dargestellt sind und die zum Erhalt einer Xho-I-Schnittstelle eingefügten Nukleotide unterstrichen dargestellt sind.
Oligonukleotid postrad_antisense (SEQ ID NO:18): Position 18 - 51 in der genomischen Sequenz, wobei die aus klonierungstechnischen Gründen angefügten Nukleotide kursiv dargestellt sind und die zum Erhalt einer *Kpn*-I-Schnittstelle eingefügten Nukleotide unterstrichen dargestellt sind.
Oligonukleotid prerad_sense SEQ ID NO:19): Position 4378 - 5357 in der genomischen Sequenz, wobei die aus klonierungstechnischen Gründen angefügten Nukleotide kursiv dargestellt sind und die zum Erhalt einer Not I Schnittstelle eingefügten Nukleotide unterstrichen dargestellt sind.
Oligonukleotid prerad_antisense (SEQ ID NO:20): Position 3084 - 3106 in der genomischen Sequenz, wobei die aus klonierungstechnischen Gründen angefügten Nukleotide kursiv; die zum Erhalt einer *Bam*-HI-Schnittstelle eingefügten Nukleotide unterstrichen und das Startcodon des *RAD54* Gens (als inverses Komplement) fett gedruckt dargestellt sind.
Oligonukleotid pma1-158_antisense (SEQ ID NO:21): entspricht Position 170 - 157 in der 939 bp deponierten Sequenz DNA des "promotor binding protein" Gens (Genbank Accession YSCHATPASA, M25502).
Oligonukleotid LEU2int_antisense (SEQ ID NO:22): entspricht Position 92511 - 92489 in der 316613 bp deponierten DNA Sequenz des LEU2 Gens (Genbank Accession NC_001135, GI:10383748, *Saccharomyces cerevisiae* chromosome III, complete chromosome sequence).
Oligonukleotid prerad_sense_int1 (SEQ ID NO:23): Position 4503 - 4481 in der *RAD54* Gensequenz (Genbank Accession SCYGL163C, Z72685, Y13135),
Oligonukleotid ura3_antisense (SEQ ID NO:24): Position 267 - 245 in der URA3 Gensequenz (Genbank Accession 406851).

### Beispiel 1: Konstruktion des Dsred Integrationsplasmids zur zytotoxischen Signalgebung

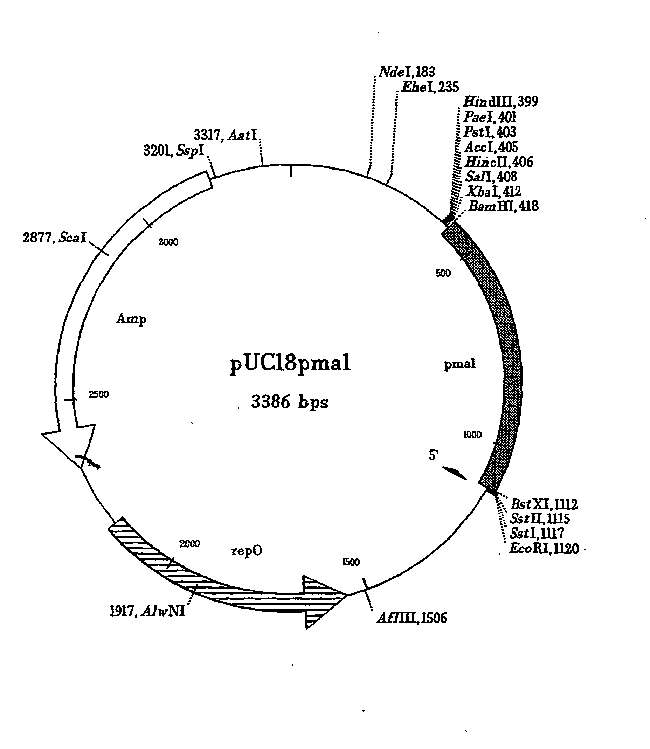

Konstruktion des Plasmids p774pma1Dsred: Zur Transkription des "Red-fluorescent-protein"-Gens in der Hefe *Saccharomyces cerevisiae* wurde der hefe-eigene Promotor der Plasmamembran ATPase *PMA1* verwendet. Der in *Saccharomyces cerevisiae* konstitutiv aktive *pma1* Promotor wurde als *Eco* RI/*Bam* HI 0.93 kb Fragment aus dem Plasmid pRS408 (erhalten von Dr. A. Goffeau, Université Catholique de Louvain-la-Neuve, Belgien) nach Auftrennung durch Agarosegelelektrophorese isoliert. In einer Ligation wurde das 0.93 kb *pma1 Eco* RI/*Bam* HI Fragment mit dem *Eco* RI/*Bam* HI gespaltenem Plasmidvektor pUC18 ligiert. Das erhaltene Plasmid pUC18-pma1 (s. Fig. 1) wurde durch Restriktionskartierung und Sequenzierung bestätigt.

Aus dem Plasmid pDsRed1-N1 (Clontech; SEQ ID NO:16) wurde das "Red fluorescent protein" Gen (Start Codon 679-681, Stop Codon 1357-1359) mit der Restriktionsendonuklease *Not* I an Position 1361 gespalten, das linearisierte Plasmid als 4.7 kb Fragment in einer Agarosegelektrophorese aufgetrennt und aus der aus der Gelmatrix eluiert. Die durch den Restriktionsverdau überstehenden Nukleotide der *Not* I Schnittstelle wurden in einer anschließenden DNA-Polymerase Enzym-Reaktion (Klenowfragment) mit 0.1 mM freien Nukleotiden (dNTPs) in 5'>3' Richtung zum Erhalt glatter Enden aufgefüllt. In einem weiteren Schritt wurde das lineare 4.7 kb pDsRed1-N1 Fragment mit der Restriktionsendonuklease *Bam* HI an Position 661 gespalten. Durch Agarosegelektrophorese wurde das *Bam* HI/*(Not* I aufgefüllt) 0.7 kb Fragment mit dem "Red-fluorescent-protein"-Gen aufgetrennt und aus der aus der Gelmatrix eluiert. Dieses Fragment wurde mit dem pUC18-pma1 *Bam* HI/*Hinc* II gespaltenem Vektor ligiert, in Bakterien (*E*. *coli* XL1-Blue, Stratagene) transformiert, und die nach Inkubation bei 37° C erhaltenen Kolonien analysiert. Durch Sequenzierung sowie eine *Eco* RI/*Hind* III Restriktionskartierung isolierter Plasmid-DNA und nachfolgender Auftrennung in der Agarosegelektrophorese wurde das 1,63 kb *pma1-DsRed1* Verbundfragment bestätigt.

Das pUC18-pma1-Ds-Red1 Plasmid wurde mit der Restriktionsendonuklease *Sac* I in der Polylinkerregion stromaufwärts des kombinierten *pma1-DsRed1* Fragments gespalten, als lineares 4,31 kb Fragment in einer Agarosegelektrophorese aufgetrennt und aus der Gelmatrix eluiert. Die durch den Restriktionsverdau überstehenden Nukleotide der *Sac* I Schnittstelle wurden in einer anschließenden DNA-Polymerase Enzym-Reaktion (Klenowfragment) mit 0.1 mM freien Nukleotiden (dNTPs) in 5'>3' Richtung zum Erhalt glatter Enden aufgefüllt. In einem weiteren Schritt wurde das lineare 4.31 kb pUC18-pma1-Ds-Red1 Fragment mit der Restriktionsendonuklease *Hind* III in der Polylinkerregion stromabwärts des kombinierten *pma1-DsRed1* Fragments gespalten und dieses als 1,63 kb Fragment durch Agarosegelektrophorese aufgetrennt und aus der Gelmatrix isoliert.

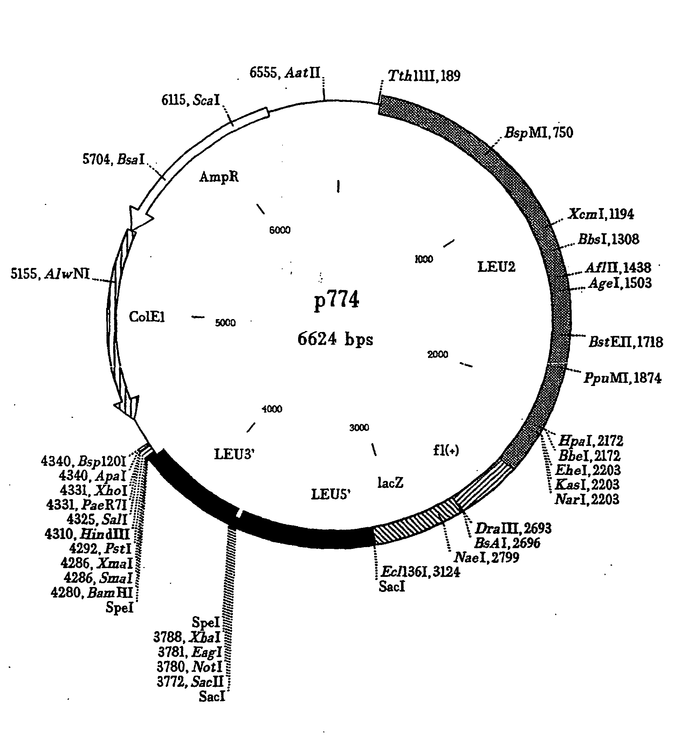

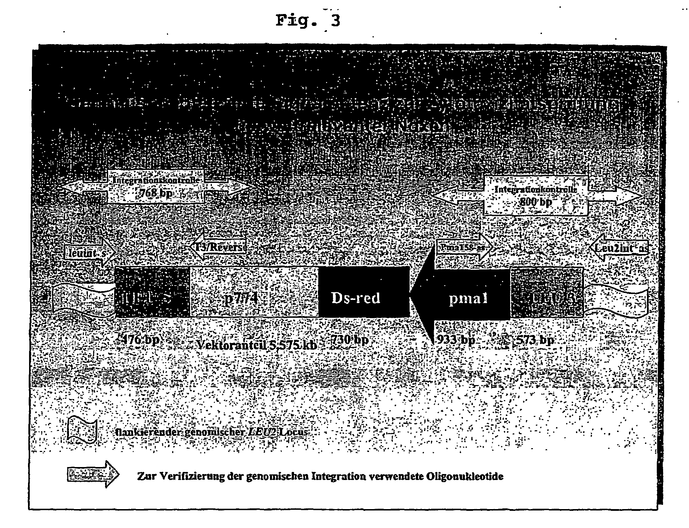

Das Plasmid p774 (Connelly & Heiter (1996) Cell 86, 275-285; erhalten von Dr. P. Ljungdahl, Ludwig Institute of Cancer Research, Stockholm, Schweden; s. Fig. 2) wurde mit den Restriktionsendonukleasen *Sma* I/*Hind* III gespalten. Dieser lineare 6,6 kb Vektor wurde mit dem 1,63 kb *pma1-Ds-Red1* Verbundfragment ligiert, in Bakterien transformiert und die nach Inkubation bei 37° C erhaltenen Kolonien analysiert. Durch eine *Bam* HI/*Sal* I Restriktionskartierung isolierter Plasmid-DNA und nachfolgender Auftrennung in der Agarosegelektrophorese wurde durch den Erhalt dreier Fragmente (6.6 kb p774, 0.93 kb *pma1* Promotor, 0,7 kb "Red-fluorescent-protein"-Gen) die Klonierung des kombinierten 1,63 kb *pma1-DsRed1* Verbundfragments in p774 bestätigt. In Figur 3 ist eine schematische Darstellung des Plasmidkonstruktes welches zur Integration der zytotoxischen Signalgebung verwendet wurde dargestellt. Mit Hilfe des Vektors p774-pma1-DsRed1 wurde das "Red-fluorescent-protein"-Gen unter Kontrolle des hefe-eigenen ATPase*pma1*-Promotors stabil in den Genlocus für den biosynthetischen Marker LEU2 des *Saccharomyces cerevisiae* Hefewirtsstamms integriert.

### Beispiel 2: Konstruktion der Rad54/gfp/URA3 Integrationskassette zur genotoxischen Signalgebung

Konstruktion des Plamids pBSK-rad54-gfp-URA3: Zur Integration der genotoxischen Signalgebung wurde zunächst ein 1,841 kb DNA Fragment, bestehend aus dem Gen für das "Green fluorescent protein" in der "yeast enhanced version" - *egfp -* mit nachfolgendem selektierbarem biosynthetischem *Saccharomyces cerevisiae* URA3 Marker-Gen, kodierend für die Orotidine-5'-phosphate Decarboxylase aus dem Plasmid pBSK-tok-*egfp-URA3* (erhalten von Dr. Jost Ludwig, MNF, Universität Tübingen) durch Spaltung mit den Restriktionsendonukleasen *Bam* HI und *Xho* I und Auftrennung in der Agarosegelektrophorese isoliert. Dieses Fragment wurde mit dem *Bam* HI und *Xho* I gespaltenem Plasmidvektor pBSKII (Stratagene) ligiert, in Bakterien (*E*. *coli* XL1-Blue, Stratagene) transformiert und die nach Inkubation bei 37° C auf LB-Amp (Luria-Bertani-Medium, 100µg/ml Ampicillin) Agarplatten erhaltenen Kolonien analysiert. Durch *Bam* HI/*Xho* I Restriktionskartierung isolierter Plasmid-DNA wurde das inserierte *egfp-URA3* Fragment (1,84 kb) in pBSKII (2,96 kb) bestätigt.

Aus dem *Saccharomyces cerevisiae* Wildstamm S288C (ATCC, USA) wurde genomische DNA mit Standardmethoden isoliert. 20 pg dieser chromosomalen DNA wurden für eine Polymerase-Kettenreaktion (PCR) mit DNA-Polymerase aus *Thermophilus aquaticus* (MBI Fermentas) und den Oligonukleotidprimern postrad_sense und postrad_antisense zur Amplifikation der 3' nicht-kodierenden Region des *S*. *cerevisiae RAD54* Gens eingesetzt.
Die durch die PCR amplifizierte DNA wurde als 0,4 kb Fragment durch Agarosegelektrophorese aufgetrennt und aus der Gelmatrix isoliert. Diese DNA wurde mit den Restriktionsendonukleasen *Kpn* I/*Xho* I gespalten, durch Agarosegelektrophorese aufgetrennt, aus der Gelmatrix isoliert und mit dem *Kpn* I/*Xho* I linearisierten Plasmidvektor pBSK-egfp-URA3 (4,82 kb) ligiert. Nach Transformation in Bakterien (*E*. *coli* XL1-Blue, Stratagene) und Inkubation bei 37° C auf LB-Amp (Luria-Bertani-Medium, 100 µg/ml Ampicillin, [Sambrook, J., Fritsch, E.F. and Maniatis, T. (1989). In: Molecular cloning: A laboratory manual. Cold Spring Harbor Laboratory Press, Cold Spring Harbor, New York]) Agarplatten wurden die erhaltenen Kolonien analysiert. Durch eine *Xho* I/*Kpn* I Restriktionskartierung isolierter Plasmid DNA und Auftrennung im Agarosegel wurde das 2,26 kb *egfp-URA3-postrad54* Verbundfragment in pBSKII (2,96 kb) bestätigt.

Aus dem *Saccharomyces cerevisiae* Wildstamm S288C (ATCC, USA) wurde genomische DNA mit Standardmethoden isoliert. 20 pg dieser chromosomalen DNA wurden für eine Polymerase-Kettenreaktion (PCR) mit DNA-Polymerase aus *Thermophilus aquaticus* (MBI Fermentas) und den Oligonukleotidprimern prerad_sense und prerad_antisense zur Amplifikation der 5' nicht-kodierenden Region des *S*. *cerevisiae RAD54* Gens plus des Startcodons und eines weiteren eingesetzt.

Das durch die PCR amplifizierte DNA-Fragment wurde als 1,3 kb Fragment durch Agarosegelektrophorese aufgetrennt und aus der Gelmatrix isoliert. Diese DNA wurde mit den Restriktionsendonukleasen *Bam* HI/*Not* I gespalten, durch Agarosegelektrophorese aufgetrennt, aus der Gelmatrix isoliert und mit dem *Bam* HI/*Not* I linearisierten 5,2 kb Plasmidvektor pBSK-*egfp-URA3-postrad54* ligiert. Nach Transformation in Bakterien *(E. coli* XL1-Blue, Stratagene) und Inkubation bei 37 °C auf LB-Amp (Luria-Bertani-Medium, 100 µg/ml Ampicillin) Agarplatten wurden die erhaltenen Kolonien analysiert. Durch eine *Not* I/*Kpn* I Restriktionskartierung isolierter Plasmid DNA und Auftrennung im Agarosegel wurde das 3,5 kb *prerad54-egfp-URA3-postrad54* Verbundfragment in pBSKII (2,96 kb) bestätigt.

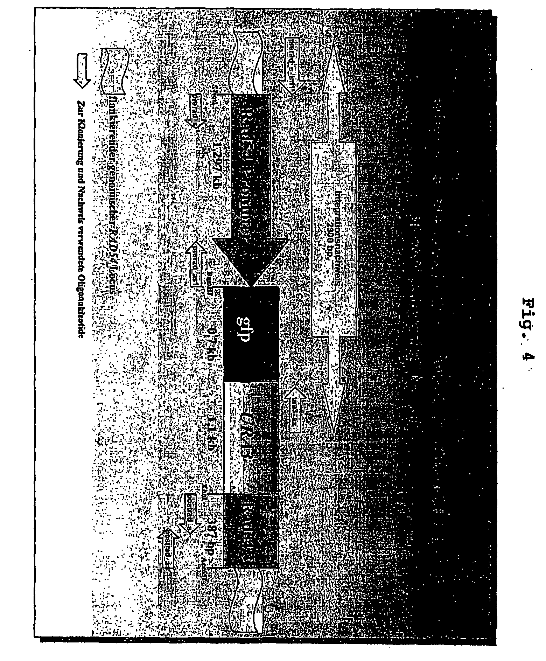

In Figur 4 ist eine schematische Darstellung des zur Integration in das *S*.*cerevisiae*-Genom verwendeten *prerad54-egfp-URA3-postrad54* DNA Konstrukts gegeben. Mithilfe der *rad54* nicht-kodierenden Sequenzen dieser Kassette wurde das *egfp-URA3* Verbundfragment zielgerichtet durch homologe Rekombination stabil in den Genlocus des *S*. *cerevisiae-RAD54*-Gens auf Chromosom VII integriert. Dadurch ist der gesamte *RAD54* kodierende Leserahmen durch das *egfp-URA3* Verbundfragment ersetzt. Die nicht gentechnisch veränderten *RAD54* Promotor Steuerelemente regulieren die Expression des "Green-fluorescent-protein"-*egfp*-Gens.

### Beispiel 3: Konstruktion des Saccharomyces-cerevisiae-Stammes mit integrierter zytotoxischer und genotoxischer Signalgebung

3.1. Konstruktion des pma1-DsRed1 und rad54-gfp exprimierenden Saccha *romyces-cerevisiae*-Stammes: In dem Plasmid p774-*pma1-DsRed1* (siehe Beispiel 1, Fig. 3) sind zur gerichteten homologen Rekombination des klonierten Inserts in den chromosomalen Locus des *LEU2* Gens (Chromosom III) in *Saccharomyces cerevisiae* 5' 476 bp und 3' 573 bp als flankierende Regionen des *LEU2* Gens inseriert. Zur gerichteten Integration des Gesamtplasmids einschließlich des 1,63 kb *pma1-DsRed1* Verbundfragments am chromosomalen Locus des *LEU2* Gens wurde mit der Restriktionsendonuklease *Not* I eine Linearisierung des Plasmids p774-*pma1-DsRed1* an Position 3780 (bezogen auf Originalplasmid, siehe Fig. 2), zwischen den flankierenden *LEU2* Regionen eingeführt.

Das erhaltene lineare 8,25 kb *Not* I DNA Fragment wurde nach Auftrennung durch Agarosegelelektrophorese und Isolierung aus der Gelmatrix zur Transformation des *pdr5yor1snq2* dreifach-mutanten *Saccharomyces*-cerevisiae-Hefestammes, in dem die hauptsächlichen hefe-eigenen, für die endogene Resistenz verantwortlichen ABC-Transporter Gene für Xenobiotikatranslokations-Systeme spezifisch deletiert sind, verwendet. Dazu wurden aus Einzellen stammende Kolonien (Hefe-Transformanten) auf den in dem Plasmid p774 ebenfalls enthaltenen biosynthetischen Marker LEU2 (LEU2⁺ Protrophie) selektioniert.

Die Transformation des *pdr5yor1snq2* dreifach mutanten *Saccharomyces-cerevisiae*-Hefestammes mit dem *DsRed1* Gen unter Kontrolle des *pma1* Promotors führte zur Isolierung eines *Saccharomyces-cerevisiae-Hefewirts* stamms, in dem die hauptsächlichen hefe-eigenen für die endogene Resistenz verantwortlichen ABC-Transporter Gene für Xenobiotikatranslokations-Systeme spezifisch deletiert sind und das *DsRed1* Gen am chromosomalen Locus des biosynthetischen *LEU2* Gens stabil integriert und unter Kontrolle des hefe-eigenen *pma1* Promotors exprimiert ist (Genotyp *MATa ura3-52 trp1-Δ63 leu2-Δ1 his3-Δ200 GAL2*^{*+*} *pdr5-Δ1::hisG snq2::hisG yor1-1::hisG leu2::pma1-DsRed1 LEU2).* Bei sich mitotisch vermehrenden Zellen wird das Konstrukt zur zytotoxischen Signalgebung auf die Nachkommenschaft vererbt. Die Expression des *DsRed1* Gens wird nach Anzucht einer Hefekultur durch eine rote Fluoreszenz nach spektraler Anregung bei 558 nm mit einem Emissionsmaximum bei 583 nm sichtbar.

In dem Plasmid *pBSKII-prerad54-egfp-URA3-postrad54* (siehe Beispiel 2, Fig. 4) sind zur gerichteten homologen Rekombination des klonierten Inserts *egfp-URA3* am chromosomalen Locus des *RAD54* Gens (Chromosom VII) in *S*. *cerevisiae* 5' 1,3 kb flankierende *prerad54* und 3' 0,4 kb flankierende *postrad54* Regionen inseriert. Zur gerichteten Integration der *prerad54-egfp-URA3-postrad54* Kassette am chromosomalen Locus des *RAD54* Gens wurde mit den Restriktionsendonukleasen *Not* I/*Pvu* II ein 3,62 kb Fragment aus dem Plasmid pBSKII-*prerad54-egfp-URA3-postrad54* gespalten.

Das erhaltene 3,62 kb *prerad54-egfp-URA3-postrad54* Fragment wurde nach Auftrennung durch Agarosegelektrophorese und Isolierung aus der Gelmatrix zur Transformation des Hefestammes mit zytotoxischer Signalpotenz (Genotyp *MATa ura3-52 trp1-Δ63 leu2-Δ1 his3-Δ200 GAL2*^{*+*} *pdr5-Δ 1::hisG snq2::hisG yor1-1::hisG leu2::pma1-DsRed1 LEU2)* verwendet. Dazu wurden aus Einzellen stammende Kolonien (Hefe-Transformanten) auf den in der Kassette enthaltenen biosynthetischen Marker URA3 (URA3⁺ Protrophie) selektioniert.

Die Transformation des *pdr5yor1snq2* dreifach-mutanten *Saccharomycescerevisiae*-Hefe-stammes mit integriertem *DsRed1* Gen unter Kontrolle des *pma1* Promotors mit der *prerad54-egfp-URA3-postrad54* Kassette führte zur Isolierung des *Saccharomyces cerevisiae* Hefewirtsstamms HLYSRG-12B2, in dem
1. die hauptsächlichen hefe-eigenen für die endogene Resistenz verantwortlichen ABC-Transporter Gene für Xenobiotikatranslokations-Systeme spezifisch deletiert sind, und
2. das *DsRed1*-Gen am chromosomalen Locus des biosynthetischen *LEU2* Gens stabil integriert und unter Kontrolle des hefe-eigenen *pma1* Promotors zur zytotoxischen Signalgebung exprimiert ist, und
3. das *egfp*-Gen am chromosomalen Locus des *RAD54* Gens stabil integriert und unter Kontrolle des hefe-eigenen *rad54* Promotors zur genoptoxischen Signalgebung exprimiert ist (Genotyp *MATa ura3-52 trp1-Δ63 leu2-Δ1 his3-Δ 200 GAL2*^{*+*} *pdr5-Δ1::hisG snq2::hisG yor1-1::hisG leu2::pma1-DsRed1 LEU2 rad54::egfp-URA3).* Anstelle des RAD54 Gens wird in diesem *Saccharomyces-cerevisiae-Stamm* ausschließlich das *egfp* Gen exprimiert. Bei sich mitotisch vermehrenden Zellen werden beide Konstrukte zur zytotoxischen und genotoxischen Signalgebung auf die Nachkommenschaft vererbt. Die Expression des *egfp* Gens wird nach Anzucht einer Hefekultur und Induktion der biochemischen, "RAD54" vermittelten Signaltransduktionskaskade durch eine grüne Fluoreszenz nach spektraler Anregung bei 488nm mit einem Emissionsmaximum bei 508 nm sichtbar.

3.2 Charakterisierung des pma1-DsRed1 und rad54-gfp exprimierenden *Saccharomyces-cerevisiae-*Stammes durch PCR-Analyse: Die korrekte Integration des p774-*pma1-DsRed1* Plasmids und der *egfp-URA3* Kassette am jeweiligen chromosomalen *LEU2* bzw. *RAD54* Locus des *Saccharomyces-cerevisiae*-Genoms, Genotyp *MATa ura3-52 trp1-Δ63 leu2-Δ1 his3-Δ200 GAL2*^{*+*} *pdr5-Δ1::hisG snq2::hisG yor1-1::hisG leu2::pma1-DsRed1 LEU2 rad54::egfp-URA3* wurde mit PCR Analysen nachgewiesen.

Als Matrize wurde von der mit Standardmethoden isolierten genomischen DNA aus vier verschiedenen selektionierten Hefe-Einzelkolonien 20 pg eingesetzt.
Die korrekte Integration des p774-*pma1-DsRed1* Plasmids am chromosomalen *LEU2*-Locus wurde mit einem Oligonukleotid, welches komplementär zum kodierenden Strang des inserierten *pma1* Promotors ist, in "sense" Richtung, und einem Oligonukleotid, welches stromabwärts außerhalb der inserierten flankierenden LEU2 3'-Region komplementär zum kodierenden Strang ist, in "antisense" Richtung (Primer pma1-158_antisense und Leu2int_antisense) nachgewiesen.

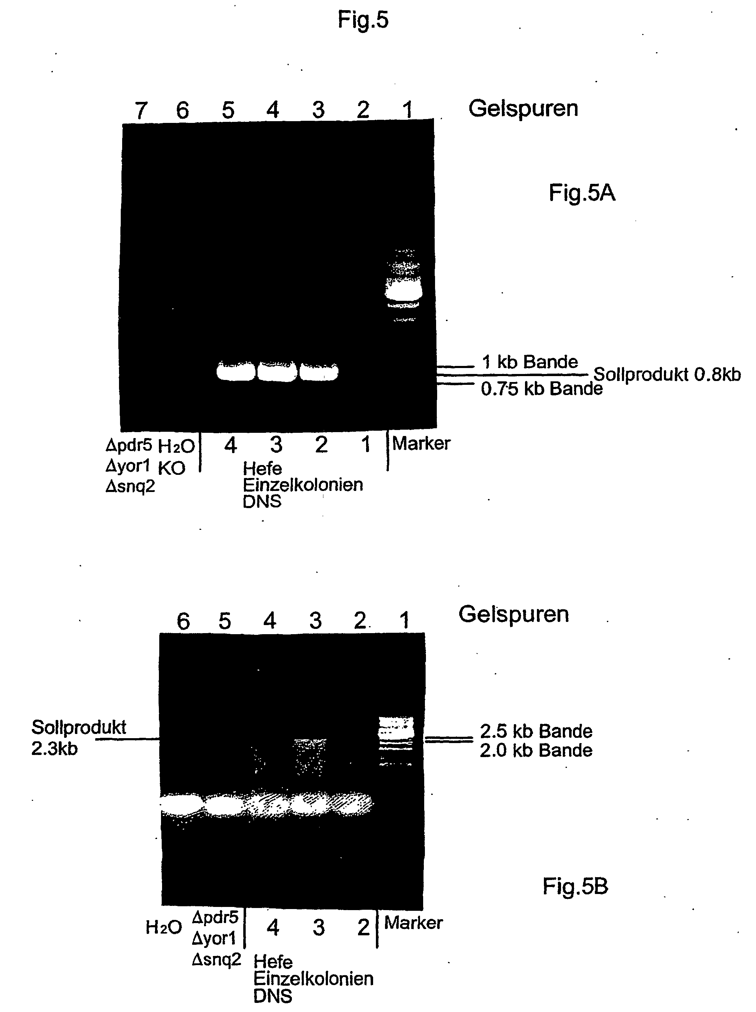

Dadurch sollte ein 0,8 kb spezifisches DNA Fragment amplifiziert werden. Zur Kontrolle des Reaktionsgemisches wurde eine Reaktion ohne Matrize durchgeführt (Wasserkontrolle, Gelspur 7 in Fig. 5A). Zur Kontrolle des korrekten Amplifikationsproduktes wurde 20 pg des *pdr5yor1snq2* dreifach mutanten *Saccharomyces-cerevisiae*-Ausgangsstammes eingesetzt (Negativkontrolle, Gelspur 6 in Fig. 5A). In Fig. 5A sind die Reaktionsansätze mit der isolierten DNA von vier verschiedenen Hefe-Einzelkolonien durch Agarosegelektrophorese aufgetrennt. Gelspur 1 enthält den Molekulargewichtsmarker (Nr. VII, MBI Fermentas), Gelspur 2 den Reaktionsansatz des analysierten Hefe-Kions 1, der keine spezifische Amplifikation erkennen lässt, Gelspuren 3 - 5 die Reaktionsansätze der analysierten Hefe-Klone 2, 3 und 4, die die spezifische Amplifikation des gewünschten Sollproduktes von 0,8 kb zeigen und damit die erfolgreiche Integration des p774-*pma1-DsRed1* Plasmids am chromosomalen *LEU2*-Locus in den Hefe-Einzelkolonien 2, 3 und 4 bestätigen.

Die korrekte Integration der *egfp-URA3* Kassette am chromosomalen *RAD54* Locus des *Saccharomyces-cerevisiae-Genoms* wurde mit der DNA der zuvor bestätigten Hefe-Klone 2, 3 und 4 als Matrize und mit einem Oligonukleotid, welches komplementär zum kodierenden Strang des biosynthetischen Marker-Gens *URA3* ist, in "antisense" Richtung, und einem Oligonukleotid, welches stromaufwärts außerhalb der inserierten flankierenden *RAD54* 5' Region homolog zum kodierenden Strang ist, in "sense" Richtung (Primer prerad_sense_int1 und ura3_antisense) nachgewiesen.

Dadurch sollte ein 2,3 kb spezifisches DNA-Fragment amplifiziert werden. Zur Kontrolle des Reaktionsgemisches wurde eine Reaktion ohne Matrize durchgeführt (Wasserkontrolle, Gelspur 6 in Fig. 5B). Zur Kontrolle des korrekten Amplifikationsproduktes wurde 20 pg des *pdr5yor1snq2* dreifach mutanten *Saccharomyces-cerevisiae*-Ausgangsstammes eingesetzt (Negativkontrolle, Gelspur 5 in Fig. 5B). In Fig. 5B sind die Reaktionsansätze mit der isolierten DNA der zuvor bestätigten Hefe-Einzelkolonien 2, 3 und 4 durch Agarosegelektrophorese aufgetrennt. Gelspur 1 enthält den Molekulargewichtsmarker (Nr. VII, MBI Fermentas), Gelspuren 2 - 4 die Reaktionsansätze der analysierten Hefe-Klone 2, 3 und 4, von denen nur der Hefe-Klon 3 eine spezifische Amplifikation des gewünschten Sollproduktes von 2,3 kb zeigt und damit die erfolgreiche Integration der *egfp-URA3* Kassette am chromosomalen *RAD54* Locus des *Saccharomyces cerevisiae* Genoms des Hefe-Klons 3 bestätigt.

Diese isolierte Hefe-Einzelkolonie mit dem Genotyp Genotyp *MATa ura3-52 trp1-Δ63 leu2-Δ1 his3-Δ200 GAL2*^{*+*} *pdr5-Δ1::hisG snq2::hisG yor1-1::hisG leu2::pma1-DsRed1 LEU2 rad54::egfp-URA3* erhielt die Bezeichnung HLY5RG-12B2 und wurde am 5.12.00 als Glycerinkultur bei der DSMZ - Deutsche Sammlung von Mikroorganismen und Zellstrukturen GmbH, Mascheroder Weg 1b, 38124 Braunschweig, Deutschland, gemäß der Richtlinien des Budapester Vertrags unter der Bezeichnung DSM 13954 hinterlegt.

3.3 Charakterisierung des *DsRed1* und *egfp* exprimierenden *Saccharomyces*-*cerevisiae*-Stammes durch Wachstums- und Fluoreszenztests in Gegenwart von Inhibitoren:
Kulturen von *Saccharomyces-cerevisiae*-Wildstamm, des *pdr5yor1snq2* dreifach-mutanten Hefestammes und des zytotoxisch und genotoxisch signalgebenden, *Dsred* und *egfp* exprimierenden Stammes HLY5RG-12B2 wurden im Vollmedium YPD (2 % Hefeextrakt, 1 % Pepton) mit 2 % D-Glucose, pH 5.0 bei 30° C über Nacht unter Schütteln angezogen.

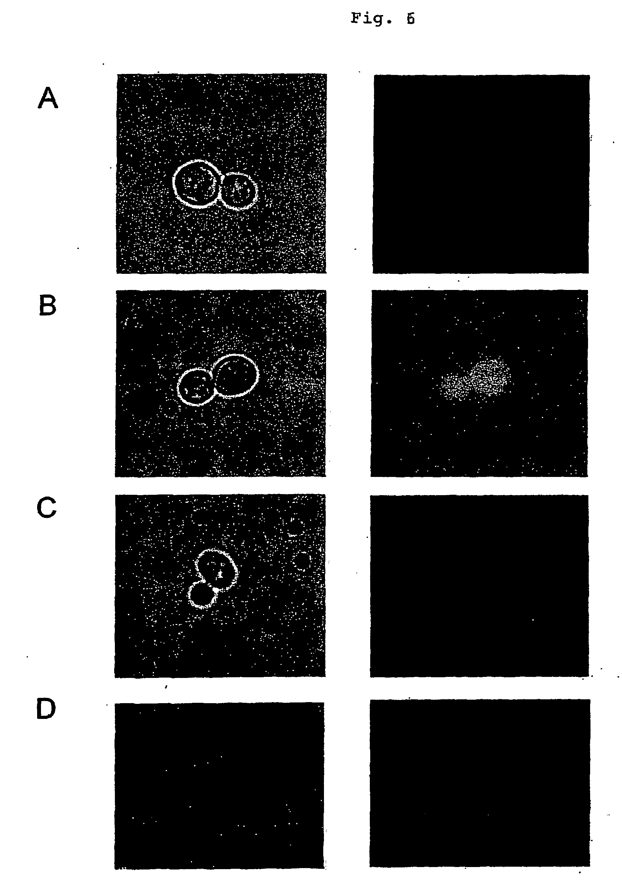

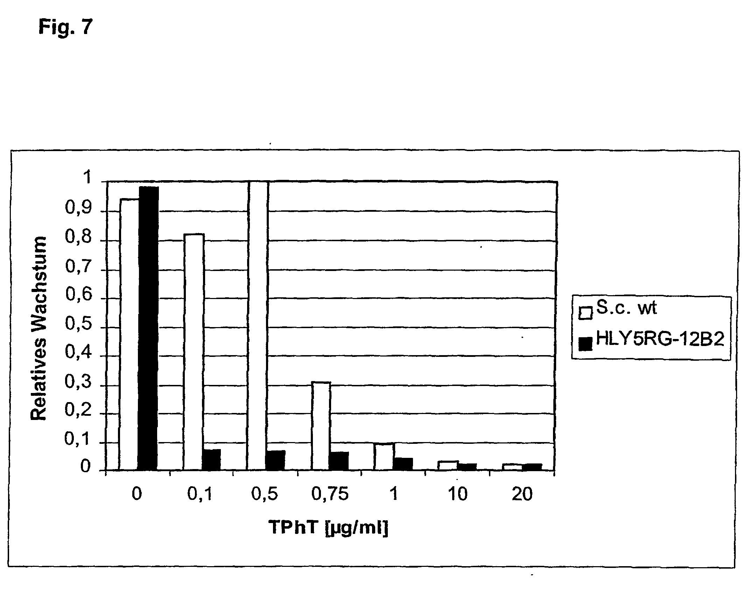

Von jedem Stamm wurden 1 x 10⁵ Zellen unter selektiven und inhibitorischen Bedingungen (0.67% yeast nitrogen base without amino acids, 0.5% NH₄SO₄, 2% D-glucose, pH 5.0 Grundmedium, für den *pdr5yor1snq2* dreifach-mutanten Hefestamm supplementiert mit Leucin, Tryptophan, Histidin und Uracil a 20 µg/l, für den Hefestamm HLY5RG-12B2 supplementiert mit Tryptophan und Histidin á 20 µg/l) mit 0.05 ng/ml und 0.5 ng/ml Mitomycin C sowie 0.01 ng/ml und 0.1 ng/ml TPhT (Tri-phenyl-Zinn) bei 30° C für 8 Stunden inkubiert. In Figur 6 sind Fluoreszenzsignale von Zellen des konstruierten und isolierten zytotoxisch und genotoxisch signalgebenden Hefestamms HLY5RG-12B2 in Abhängigkeit von der Inhibitorkonzentration im Kulturmedium gezeigt. Fig. 7 zeigt das Wachstum von *S*.*cerevisiae*-Wildtyp-Zellen und von Zellen des konstruierten Hefestamms HLY5RG-12B2 nach 8 Stunden Inkubation in Abhängigkeit von der Ihibitorkonzentration.

Der *Saccharomyces-cerevisiae*-Wildstamm wächst unter den oben genannten Bedingungen inhibitorischen Bedingungen von 0.05 ng/ml und 0.5 ng/ml Mitomycin C sowie 0.01 ng/ml und 0.1 ng/ml TPhT mit normalen Wachstumsraten (Verdopplungszeit 90 min) ohne eine spezifische rote (zytotoxisches Potential) oder grüne (genotoxisches Potential) Fluoreszenz bei Emissionsmaxima von 583 nm anzuzeigen. Aufgrund der normalen Wachstumsraten hat dieser Wildstamm eine unspezifische Hintergrundfluoreszenz durch stationäre Zellen. Der Hefestamm mit Defekten in drei Xenobiotikatranslokations-Systemen PDR5, YOR1 und SNQ2 (*pdr5yor1snq2*, Dreifach-Mutante) wächst mit niedrigen Wachstumsraten (Verdopplungszeit 180 min) ohne eine spezifische rote (zytotoxisches Potential) oder grüne (genotoxisches Potential) Fluoreszenz bei Emissionsmaxima von 583 nm bzw. 508 nm anzuzeigen. In Abhängigkeit der verwendeten Konzentration des genotoxischen Inhibitors Mitomycin C (genotoxisches Potential, Fig. 6 A und B) wurde eine zunehmende spezifische grüne Fluoreszenz, Emissionsmaxima von 508 nm, aber keine zunehmende rote Fluoreszenz, Emissionsmaxima von 583 nm, detektiert. In Abhängigkeit der verwendeten Konzentration des zytotoxischen Inhibitors TPhT (zytotoxisches Potential, Fig. 6, C und D) wurde eine zunehmende spezifische rote Fluoreszenz, Emissionsmaxima von 583 nm, aber keine zunehmende grüne Fluoreszenz, Emissionsmaxima von 508 nm, detektiert.

Damit wurde die selektive Detektion zytotoxischen und genotoxischen Potentials verschiedener Substanzen im S. *cerevsiaie* Stamm HLY5RG-12B2 bestätigt.

### Beispiel 4: Zytotox- und Genotox-Testverfahren unter Verwendung von HLY5R

Zur zyto- und genotoxischen Substanzprüfung wurde eine Flüssig-Vorkultur des *Saccharomyces-cerevisiae*-Hefestammes HLY5RG-12B2 in 5 ml Volumen, bestehend aus YNB Medium (1,7 g/l "Yeast Nitrogen Base" ohne Aminosäuren), 5 g/l NH₄SO₄, 2% D-Glucose, 0,5g/l Aminosäure-Mix (bestehend aus: 250 mg Adenin, 500 mg Tryptophan, 100 mg Arginin, 100 mg Methionin, 150 mg Tyrosin, 150 mg Lysin, 300 mg Valin, 500 mg Threonin, 500 mg Serin, 250 mg Phenylalanin, 100 mg Asparagin, 10 mg Glutaminsäure, 100 mg Histidin)), pH 5.9 bei 30° C über Nacht (12 - 18 h) unter Schütteln (180 rpm) angezogen. Die Zellen befanden sich dann in der logarithmischen Wachstumsphase, ein Aliquot wurde mikroskopisch kontrolliert und die Zellzahl ausgezählt.

Von diesem vorkultivierten Stamm wurden 1 x 10⁵ Zellen/ml zum Test im gleichen Medium inokkuliert. Für die genotoxischen Substanzprüfungen wurde eine Eichreihe mit vier verschiedenen Konzentrationen Mitomycin C, beginnend bei 0.05 ng/ml und endend bei 0.5 ng/ml angelegt. Für die zytotoxischen Substanzprüfungen wird eine Eichreihe mit vier verschiedenen Konzentrationen TPhT (Tri-phenyl-Zinn), beginnend bei 0.01 ng/ml und endend bei 0.1 ng/ml angelegt.

Von zu prüfenden wässrigen Lösungen wurden Verdünnungen in absteigender Konzentration in 10er Schritten in geeignetem Lösungsmittel hergestellt (mindestens 10 pro zu prüfender Lösung) und zu den vorgelegten Zellen in Kultivierungsröhrchen (1 - 10 ml) oder Mikrotiterschalen-Kavitäten (50 - 200 µl) gegeben.

Von zu prüfenden Festsubstanzen wurden definierte Mengen abgewogen (ng - µg - mg Bereich) und zu den vorgelegten Zellen in Kultivierungsröhrchen (1 - 10 ml) oder Mikrotiterschalen-Kavitäten (50 - 200 µl) gegeben, oder definierte Lösungen (in Prozent oder in g/l) in geeignetem Lösungsmittel hergestellt und ebenfalls zu der vorgelegten Zellsuspension gegeben.
Zur Kontrolle wurde eine Anzahl Kultivierungsröhrchen (1 - 10 ml) oder Mikrotiterschalen-Kavitäten (50 - 200 µl) mit vorgelegten Zellen ohne jede zu prüfende Substanz oder Lösung pipettiert.
Alle so vorbereiteten Kultivierungsröhrchen (1 - 10 ml) oder Mikrotiterschalen-Kavitäten (50 - 200 µl) wurden für 8 Stunden bei 30° C unter Schütteln (180 rpm) inkubiert.

Anschließend wurde für die genotoxische Substanzprüfung eine spektrale Anregung bei 489nm durchgeführt und die Intensität der Emissionsmaxima bei 508 nm (spezifisch für *egfp)* aller Zellsuspensionen gemessen. Diese Daten wurden protokolliert und ausgewertet. unter Einbeziehung der entsprechenden Eichreihe sowie der Kontrollreihe.

Für die zytotoxische Substanzprüfung wurde eine spektrale Anregung bei 558nm durchgeführt und die Intensität der Emissionsmaxima bei 583 nm (spezifisch für *DsRedp)* aller Zellsuspensionen gemessen. Außerdem wurde eine Bestimmung des einfachen Wachstums der Zellen durch Messung der optischen Dichte bei 600 nm durchgeführt. Diese Daten wurden protokolliert und ausgewertet unter Einbeziehung der entsprechenden Eichreihe sowie der Kontrollreihe.

## Patentansprüche

1. Modifizierter Hefestamm in dem
(1) eine Genotox-Kassette, umfassend einen ersten Promoter, der durch genotoxische Agentien induzierbar ist, und ein erstes Reportergen, das funktional mit dem ersten Promoter verknüpft ist, und
(2) eine Zytotox-Kasette, umfassend einen zweiten Promoter, der durch zytotoxische Agentien induzierbar ist, und ein zweites Reportergen, das funktional mit dem zweiten Promoter verknüpft ist,
wobei die Promoter und Reportergene in (1) und (2) voneinander verschieden sind,
stabil und funktional in das Genom eines Hefewirtsstammes integriert sind.

2. Hefestamm nach Anspruch 1, wobei der Hefewirtsstamm aus der Gattung *Ascomycota* ist und insbesondere ein *Saccharomyces-cerevisiae-Stamm* ist.

3. Hefestamm nach Anspruch 1 oder 2, wobei der Hefestamm durch Disruption oder Deletion einer oder mehrerer der in dem Hefewirtsstamm vorhandenen Xenobiotikatranslokationsgene sensibilisiert ist.

4. Hefestamm nach Anspruch 1, wobei die Xenobiotikatranslokationsgene ausgewählt sind aus PDR5, YOR1, SNQ2, YCF1, PDR10, PDR11 und PDR12 und insbesondere wenigstens das PDR5-Gen, besonders bevorzugt das PDR5-, YOR1- und SNQ2-Gen deletiert ist.

5. Hefestamm nach einem oder mehreren der Ansprüche 1 bis 4, wobei
(i) der erste Promoter ein Promotor ist, der durch genotoxische Agentien induziert wird und Reparaturmechanismen ansteuert, die als Folge von primären DNS-Schäden aktiviert werden, vorzugsweise ein Promotor zur Regulation von Gen- oder Zellreparaturgenen ist und besonders bevorzugt ein Promoter der Rad-Gene oder der Heat-Schock-Gene ist; und/oder
(ii) der zweite Promoter ein Promotor ist, der die konstitutive Expression von Haushaltsgenen reguliert und durch zytotoxische Agenzien deaktiviert wird, und insbesondere ein Promoter eines Tubulins oder eines Stoffwechselenzyms ist; und/oder
(iii) das erste und zweite Reportergen ausgewählt sind aus Fluoreszenzmarkern, Enzymen oder Antigenen und insbesondere zwei nicht interferierende Fluoreszenzmarker sind.

6. Hefestamm nach Anspruch 5, wobei der erste Promoter ein Rad-54-Promoter ist, das erste Reportergen ein grün-fluoreszierendes Protein, insbesondere GFP aus *Aquorea victoria* oder eine Mutante desselben ist, der zweite Promoter ein Leu2-Promoter ist und das zweite Reportergen ein rotfluoreszierende Protein, insbesondere Dsred aus *Dicostoma* oder eine Mutante desselben ist.

7. Hefestamm nach einem oder mehreren der Ansprüche 1 bis 6, wobei die Gentox-Kassette und/oder die Zytox-Kassette weiterhin noch funktionelle DNA-Sequenzen oder funktionelle Gene, insbesondere Selektions/Markergene, Rekombinase-Erkennungssequenzen und/oder Spleißstellen enthalten.

8. Hefestamm nach Anspruch 1, wobei der Hefestamm eine *Saccharomycescerevisiae*-Mutante *pdr5yor1snq2 LEU2::pma1-Dsred RAD54::gfp* ist und insbesondere HLY5RG-12B2 (DSM 13954) ist.

9. Verfahren zur Herstellung eines modifizierten Hefestamms gemäß Ansprüchen 1 bis 8, umfassend das Integrieren einer Genotox- und einer Zytotox-Kassette in einen Hefewirtsstamm.

10. Verfahren nach Anspruch 9, wobei weiterhin eines oder mehrere Xenobiotikatranslationsgene disruptiert oder deletiert werden.

11. Verfahren nach Anspruch 10, wobei die Gentox- und Zytox-Kassetten in das Genom eines *Saccharomyces cerevisiae pdr5yor1snq2* Hefewirtsstammes integriert werden.

12. Ein Verfahren zur Detektion umweltrelevanter Noxen, umfassend:
(a) die Behandlung eines modifizierten Hefestammes gemäß Ansprüchen 1 bis 8 mit einer Testsubstanz oder einem Testsubstanzgemisch;
(b) Wachstumsbestimmungen in Anwesenheit oder nach Beendigung der Behandlung mit der Testsubstanz/dem Testsubstanzgemisch, und
(c) Messungen des Anstiegs oder der Abnahme der Reportergenaktivität des Hefestammes in Anwesenheit oder nach Beendigung der Behandlung mit der Testsubstanz/dem Testsubstanzgemisch.

13. Ein Verfahren nach Anspruch 12, welches zur Detektion von genotoxischer Substanzen und/oder zytotoxischer Substanzen geeignet ist.

14. Verwendung eines modifizierten Hefestamms nach Ansprüchen 1 bis 8 zur Prüfung der Geno- und/oder Zytotoxizität komplexer Umweltkontaminationen.

15. Testkit und Biosensor zur Prüfung der Geno- und/oder Zytotoxizität komplexer Umweltkontaminationen, umfassend einen modifizierten Hefestamm nach Ansprüchen 1 bis 8.

## Claims

1. A modified yeast strain in which
(1) a genotox cassette comprising a first promoter inducible by genotoxic agents and a first reporter gene functionally linked to the first promoter; and
(2) a cytotox cassette comprising a second promoter inducible by cytotoxic agents and a second reporter gene functionally linked to the second promoter;
wherein the promoters and reporter genes in (1) and (2) are respectively distinct from each other;
are stably and functionally integrated in the genome of a yeast host strain.

2. The yeast strain according to claim 1, wherein said yeast host strain is of the phylum Ascomycota, especially being a *Saccharomyces cerevisiae* strain.

3. The yeast strain according to claim 1 or 2, wherein said yeast strain has been sensitized by disrupting or deleting one or more of the xenobiotic translocation genes present in the yeast host strain.

4. The yeast strain according to claim 1, wherein said xenobiotic translocation genes are selected from PDR5, YOR1, SNQ2, YCF1, PDR10, PDR11 and PDR12, especially wherein at least the PDR5 gene is deleted, and more preferably, the PDR5, YOR1 and SNQ2 genes are deleted.

5. The yeast strain according to one or more of claims 1 to 4, wherein:
(i) said first promoter is a promoter which is induced by genotoxic agents and controls repair mechanisms which are activated in consequence of primary DNA damage, preferably being a promoter for the regulation of gene or cell repair genes, more preferably a promoter of the Rad genes or the heat shock genes; and/or
(ii) said second promoter is a promoter which regulates the constitutive expression of household genes and is deactivated by cytotoxic agents, especially being a promoter of a tubulin or of a metabolic enzyme; and/or
(iii) said first and second reporter genes are selected from fluorescent markers, enzymes or antigens, especially being two non-interfering fluorescent markers.

6. The yeast strain according to claim 1, wherein said first promoter is a Rad54 promoter, said first reporter gene is a green fluorescent protein, especially GFP from *Aequoria victoria* or a mutant thereof, said second promoter is a Leu2 promoter, and said second reporter gene is a red fluorescent protein, especially Dsred from *Discosoma* or a mutant thereof.

7. The yeast strain according to one or more of claims 1 to 6, wherein the genotox cassette and/or the cytotox cassette further contain functional DNA sequences or functional genes, especially selectable marker genes, recombinase recognition sequences and/or splicing sites.

8. The yeast strain according to claim 1, wherein said yeast strain is a Sac*charomyces cerevisiae* mutant *pdr5yorlsnq2 LEU2::pma1-Dsred RAD54::gfp*, especially HLY5RG-12B2 (DSM 13954).

9. A method for the preparation of a modified yeast strain according to claims 1 to 8, comprising the integration of genotox and cytotox cassettes into a yeast host strain.

10. The method according to claim 9, wherein one or more xenobiotic translocation genes are further disrupted or deleted.

11. The method according to claim 10, wherein said genotox and cytotox cassettes are integrated into the genome of a *Saccharomyces cerevisiae* pdr5yor1snq2 yeast host strain.

12. A method for the detection of noxious substances relevant to the environment, comprising:
(a) the treatment of a modified yeast strain according to claims 1 to 8 with a test substance or a mixture of test substances;
(b) determinations of growth in the presence or after completion of the treatment with said test substance/mixture of test substances; and
(c) measurements of the increase or decrease of the reporter gene activity of the yeast strain in the presence or after completion of the treatment with said test substance/mixture of test substances.

13. The method according to claim 12, which is suitable for the detection of genotoxic substances and/or cytotoxic substances.

14. Use of a modified yeast strain according to claims 1 to 8 for testing the genotoxicity and/or cytotoxicity of complex environmental contaminations.

15. A test kit and biosensor for testing the genotoxicity and/or cytotoxicity of complex environmental contaminations, comprising a modified yeast strain according to claims 1 to 8.

## Revendications

1. Souche de levure modifiée, **caractérisée en ce que** sont intégrés de façon stable et fonctionnelle dans le génome d'une souche hôte de levure
(1) une cassette génotoxique contenant un premier promoteur susceptible d'être induit par des agents génotoxiques, et un premier gène reporteur lié de manière fonctionnelle au premier promoteur, et
(2) une cassette cytotoxique contenant un second promoteur susceptible d'être induit par des agents cytotoxiques, et un second gène reporteur lié de manière fonctionnelle au second promoteur,
les promoteurs et les gènes reporteurs dans (1) et (2) étant différents les uns des autres,

2. Souche de levure selon la revendication 1, dans laquelle la souche hôte de levure est du type *Ascomycota* et est en particulier une souche *Saccharomyces-cerevisiae*.

3. Souche de levure selon la revendication 1 ou 2, dans laquelle la souche de levure est sensibilisée par perturbation ou suppression d'un ou de plusieurs gènes de translocation de xénobiotiques présents dans la souche hôte de levure.

4. Souche de levure selon la revendication 1, dans laquelle les gènes de translocation de xénobiotiques sont choisis parmi PDR5, UOR1, SNQ2, YCF1, PDR10, PDR11 et PDR12 et dans laquelle en particulier au moins le gène PDR5, de préférence spécifiquement les gènes PDR5, YOR1 et SNQ2, est supprimé.

5. Souche de levure selon une ou plusieurs des revendications 1 à 4, dans laquelle
(i) le premier promoteur est un promoteur induit par des agents génotoxiques et commandant des mécanismes de réparation qui sont activés à la suite de dommages ADN primaires, de préférence un promoteur pour réguler les agents de réparation des gènes ou des cellules et de préférence particulière un promoteur des gènes Rad ou des gènes de choc thermique ; et/ou
(ii) le second promoteur est un promoteur régulant l'expression constitutive de gènes de régulation et désactivé par des agents cytotoxiques, et en particulier un promoteur de tubuline ou d'enzyme de métabolisme ; et/ou
(iii) le premier et le second gènes reporteurs sont choisis parmi les marqueurs de fluorescence, les enzymes ou les antigènes et en particulier deux marqueurs de fluorescence ne faisant pas interférence l'un avec l'autre.

6. Souche de levure selon la revendication 5, dans laquelle le premier promoteur est un promoteur Rad-54, le premier gène reporteur est une protéine présentant une fluorescence verte, en particulier GFP de *Aquorea victoria* ou un mutant de celle-ci, le second promoteur est un promoteur Leu-2 et le second gène reporteur est une protéine fluorescente rouge, en particulier Dsred de *Dicostoma* ou un mutant de celle-ci.

7. Souche de levure selon un ou plusieurs des revendications 1 à 6, dans laquelle la cassette génotoxique et/ou la cassette cytotoxique contiennent en outre d'autres séquences d'ADN fonctionnelles ou des gènes fonctionnels, en particulier des gènes sélectifs/marqueurs, des séquences de reconnaissance de recombinant et/ou des points d'épissure.

8. Souche de levure selon la revendication 1, dans laquelle la souche de levure est un mutant de *Saccharomyces-cerevisiae pdr5yor1snq2 LEU2 : :pma1-Dsred RAD54::gfp* et en particulier HLY5RG-12B2 (DSM 13954).

9. Procédé de production d'une souche de levure modifiée selon les revendications 1 à 8, contenant l'intégration d'une cassette génotoxique et d'une cassette cytotoxique dans la souche hôte de levure.

10. Procédé selon la revendication 9, dans lequel en outre un ou plusieurs gènes de translocation de xénobiotiques sont perturbés ou supprimés.

11. Procédé selon la revendication 10, dans lequel la cassette génotoxique et la cassette cytotoxique sont intégrées dans le génome d'une souche hôte de levure *Saccharomyces-cerevisiae pdr5yor1snq2*.

12. Procédé de détection de produits nocifs pour l'environnement, comportant :
(a) le traitement d'une souche de levure modifiée selon les revendications 1 à 8 avec une substance de test ou un mélange de substances de test ;
(b) des déterminations de croissance en présence ou au terme du traitement avec la substance de test/le mélange de substances de test, et
(c) des mesures de l'élévation ou de la baisse de l'activité des gènes reporteurs de la souche de levure en présence ou au terme du traitement avec la substance de test/le mélange de substances de test.

13. Procédé selon la revendication 2, convenant à la détection de substances génotoxiques et/ou de substances cytotoxiques.

14. Application d'une souche de levure modifiée selon les revendications 1 à 8 pour contrôler la génotoxicité et/ou la cytotoxicité de contaminations de l'environnement complexes.

15. Kit de test et biodétecteur pour contrôler la génotoxicité et/ou la cytotoxicité de contaminations de l'environnement complexes, comprenant une souche de levure modifiée selon les revendications 1 à 8..
